# EUROPEAN PATENT SPECIFICATION

(11) **EP 3 873 380 B1**
(45) Date of publication and mention of the grant of the patent: **12.08.2026**
(21) Application number: 19805368.8
(22) Date of filing: 30.10.2019
(51) Int. Cl.: A61F 2/00

(54) **AN INTRAURETHRAL MAGNETIC VALVE AND ASSOCIATED PARTS**
INTRAURETHRALES MAGNETVENTIL UND ZUGEHÖRIGE TEILE
VALVE MAGNÉTIQUE INTRA-URÉTRALE ET PIÈCES ASSOCIÉES

(30) Priority: 31.10.2018 US 201862753447 P; 03.12.2018 GB 201819682
(43) Date of publication of application: 08.09.2021
(73) Proprietor: Ingenion Medical Limited, London W1G 8TB (GB)
(72) Inventor: RUSSELL, Jeremy, Guildford Surrey GU3 1LZ (GB); BRACKEN, Ronald L, Monroe, Georgia 30655 (US)
(74) Representative: HGF
(86) International application number: PCT/GB2019/053075
(87) International publication number: WO 2020/089623

(56) References cited:
- EP-A1- 0 265 207
- US-A- 6 066 088
- US-A1- 2008 269 546

## Description

Embodiments relate to intraurethral valves and valve systems, systems for the placement and/or removal of intraurethral valve systems, and the like.

There is a desire, in general, to provide intraurethral valves which are capable of selectively controlling the flow of fluid therethrough. Such valves may be needed, for example, in relation to patients with urinary retention deficiencies.

An example placement of such a valve in a male patient can be seen in figure 1, which is an extract of figure 3 of US6066088 - reference numerals specifically referring only to prior art figures (figures 1 and 2) in the present description will be presented in parentheses (similar reference numerals will be used (without parentheses in the description) to refer to embodiments, where appropriate, in order to aid understanding).

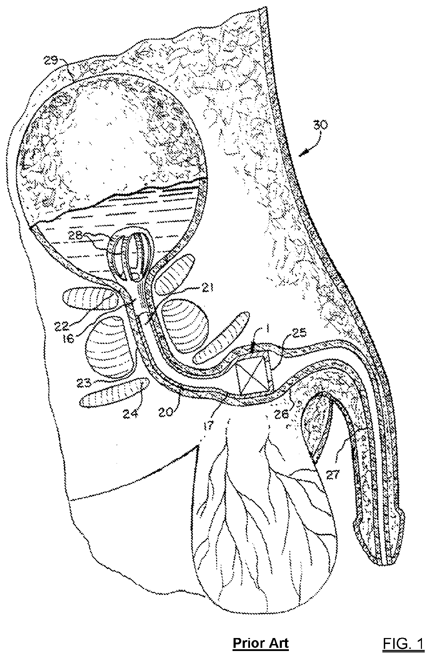

As can be seen, figure 1 shows a human male abdomen (30) in cross-section. A valve (1) may be located within a patient's bulbous urethra (26) generally at bulge (17) and generally upstream of the pendulous urethra (27) - in terms of the normal flow of fluid through the urethra. The valve (1) is coupled to a retention mechanism by a catheter (16) defining a lumen (21) - which as an outlet (25) at the valve (1) and an inlet defined by retaining loops (28) of the retention mechanism. The retention mechanism is located in the bladder (29) and includes the retaining loops (28) which hold the retention mechanism in place relative to the bladder neck (22).

The operation of the valve (1) between open and closed configurations is intended, therefore, to control the flow of fluid from the bladder (29) through the outlet (25) and to the pendulous urethra 27.

Important advances in this regard include the intraurethral magnetic valve of WO00/02499. Figure 1 of this document is repeated as figure 2 of the present application for ease of reference. The document teaches an intraurethral magnetic valve (1) for insertion in the human urethra of a person suffering from incontinence. The intraurethral magnetic valve (1) includes a nonferromagnetic cylindrical housing (2), a valve seat assembly (36) attached to one end of the housing, a spherical magnetic valve element (14) disposed for universal movement in the housing, and a ring (10) for retaining the valve element which is attached at the other end of the housing. The valve seat assembly (36) has a nonferromagnetic valve seat (4) and ferromagnetic ring (5). The valve element (14) is maintained in closed check position against the valve seat (4) by attraction to the ferromagnetic ring (5), restricting the flow of fluid through the valve (1). An external magnet is used to impart magnetic torque and attraction to the valve element (14) displacing it from the valve seat (4) and opening the valve for fluid flow. On removal of the external magnet, the valve element (14) returns to its closed check position on the valve seat (4). The document also teaches a second embodiment in which the intraurethral magnetic valve (1) includes a mechanism having a spring for relieving excessive fluid pressure, in which the valve seat assembly (36), rather than being attached to the housing (2), is axially slidable within the housing against the force of the spring when excessive hydrostatic pressure is present to allow release of fluid through a passageway around the valve seat assembly (36).

Such advanced intraurethral magnetic valves seek to allow selective release of fluid from the bladder through use of an external magnet, in other words, a magnet external to the patient's body may be brought close to the internally fitted intraurethral magnetic valve to cause the actuation thereof from a closed configured to an open configuration.

There are, however, problems with even these intraurethral magnetic valves. For example, the valves can exhibit poor sealing around the valve, between an outer part of the valve and the wall of the urethra. If there is a poor seal in this area, then the valve may operate correctly to prevent the flow of fluid therethrough unless actuated to the open configuration, but fluid may leak around the valve - particularly when the fluid pressure is relatively high. Likewise, there are problems with maintaining a good seal between the valve element and valve seat - particularly over the lifetime of the valve.

The environment in which the valve and its retention mechanism are located can result in encrustation, for example, with a mineralised biofilm of Proteus mirabilis or other microbial material. This can result in blockage of the valve or can impact the seal between the valve and the wall of the urethra (which may lead to leakage, as described above).

The placement of intraurethral valves has also been the subject of development. In particular, systems for the placement of intraurethral valves need to allow the valve and an associated retaining mechanism to be pushed through the urethra into position. However, during placement of the valve in this manner, it is not uncommon for pulling and twisting of the valve and its retaining mechanism to be required for accurate placement and navigation through the urethra. Typically such placement systems require a portion which is manipulated by the practitioner (e.g. a surgeon or other medically qualified person) and which does not, therefore, enter the patient's urethra. All or part of this portion of the placement system needs to be removable from the valve and its retaining mechanism after placement of the valve. Therefore, placement systems for intraurethral valves need to be able to navigate the valve and its retaining mechanism into position through the urethra and need to be at least partially removable following such placement.

One such example is WO2008/067557 which teaches a system for implanting a catheter in a urethra. The system includes a catheter having a magnetic intraurethral valve disposed at one end portion of the catheter. The system also includes a tool having a tip member extending into an opening at the first end portion of the catheter to couple the tool to the catheter. The system also includes an element extending from the tool. The catheter has an internal path that provides a conduit for the element to be extended through the valve to engage a second end portion of the catheter. The tool is adapted use the element to stress the catheter and stiffen the catheter along the length of the catheter Stiffening the catheter facilitates implantation of the catheter in the urethra by enabling pull forces, push forces, and torque forces.

Advancement in the area of intraurethral valve placement can also be found in WO2011032150. This document teaches an insertion facilitation device for facilitating insertion of a tube-like structure into a passage in a mammalian body. The device comprises: (a) an elongate stiffener including a proximal end portion and a distal end portion, the stiffener being adapted to extend in a lumen of the tube-like structure from a distal end portion thereof to a proximal end portion thereof, with the distal end portion of the stiffener at least partially within the distal end portion of the tube-like structure and the proximal end portion of the stiffener at least partially within the proximal end portion of the tube-like structure and provide a stiffness to the tube-like structure that is greater with the stiffener than without the stiffener, and be axially displaced within the tube-like structure relative to the tube-like structure; and (b) an engagement device that is adapted in an engaging condition thereof to releasably engage the stiffener and tube-like structure with the stiffener extending within the lumen of the tube-like structure such that either or both axial and radial displacement of the stiffener relative to the tube-like structure is substantially eliminated during insertion of the tube-like structure into a body passage, and in a disengaging condition thereof to release engagement of the stiffener and tube-like structure such that the stiffener is at least axially displaceable within the lumen of the tube-like structure and can be withdrawn from the tube-like structure at least so that the proximal end portion of the stiffener is in or adjacent the distal end portion of the tube-like structure.

These systems for the placement of intraurethral valves are, as can be seen from the aforementioned documents, relatively complex and there is a general desire to simply these systems.

There is also a desire to provide a mechanism for the removal of the valve and its retaining mechanism after placement. This may be required, for example, to enable periodic replacement of the valve and/or to allow the fitting of a conventional urinary catheter (e.g. if the patient is to be admitted into hospital for a surgical operation, drug delivery, or the like).

The advancement of intraurethral valves has concentrated on use with male patients. This is, in part, due to the longer urethra and, therefore, greater available space for the placement of such valves. There is, however, also a need for such valves in relation to female patients.

As will be appreciated, the positioning of a valve in a patient's urethra (male or female) can be uncomfortable. The valve, by necessity to form a seal, presses against the wall of the urethra - see figure 1, for example. **In** addition, external forces may increase the discomfort. For example, when a patient sits, walks, runs, or climbs stairs, then there may be additional and/or different forces acting on the valve and/or the retention mechanism. These changes in forces may alter the pressure on the urethra wall and the surrounding tissue by the valve and/or the retention mechanism. This may increase the discomfort felt by the patient (indeed, the patient may only feel discomfort when performing some activities - especially, for example, sitting).

US2008269546 discloses a self-acting urethral valve which emulates the operation of a healthy urethra by providing a valve with a large differential between the opening pressure and the closing pressure. The differential is achieved by using a snap action created by the action of the urine flow on a force that drops off rapidly with displacement. The force is achieved by using permanent magnets. The valve is installed by insertion into the urethra and can be removed without a surgical procedure. The valve is held in place by one or more grooves and ridges which abut an internal wall of the urethra.

Prior patent document EP0265207A1 describes a urethra insertable in-dwelling incontinence control device. A flexible tube has two deformable retention means affixed to the exterior of the tube near one end, and a valve bonded to the tube at the opposite end. Each of the deformable retention means is composed of a foam cuff having interconnecting interstices filled with air when the cuff is in its normally expanded condition. A lumen extending along the length of the interior surface of the flexible tube and plugged at each end communicates with the foam cuffs. A syringe can be inserted through a port into the lumen to be used to withdraw the air from the interstices in the foam cuffs.

There is a need, therefore, to improve the overall comfort of such valves when in use.

Embodiments seek to alleviate one or more problems associated with the prior art.

Accordingly, an aspect provides an intraurethral magnetic valve having an inlet port and an outlet port, the valve including: a valve main body; a ferromagnetic ring located at least partially within the valve main body; a valve seat; and a magnetic valve element located within an internal volume defined by the valve main body and configured for movement with respect to the ferromagnetic ring, that movement being within the valve main body between a closed configuration in which fluid is inhibited from flowing from the inlet port through the outlet port and an open configuration in which fluid is permitted to flow from the inlet port through the outlet port, movement of the magnetic valve element being on exposure to a magnetic field from an external magnet member, wherein the valve seat is formed from an elastomer material, wherein the valve seat is an offset valve seat such that the outlet is offset from a central longitudinal axis of the internal volume (111) defined by the valve main body..

An elastomer material of the valve main body may form the valve seat with which the magnetic valve element is configured to engage when in the closed configuration. A valve may further comprise a valve seat member defining the valve seat and fitted to the valve main body. The valve seat member may be at least partially received by the valve main body. The ferromagnetic ring may at least partially surround a portion of the valve seat member. The valve seat member may be formed of only elastomer material or materials. The ferromagnetic ring may be embedded in the elastomer material which forms the valve seat. The magnetic valve element may be configured to adopt an open pass-through configuration in which a path is provided through the valve for an extendible member. The internal valve volume may include a bulbous portion configured to receive at least part of the magnetic valve element when in the open pass-through configuration. The valve main body may be elastically deformable to reduce a width of the internal volume. The valve may further include one or more external ribs for engagement with a urethral wall. The valve may further include an external seal member configured to surround at least a part of the valve main body to aid in providing a seal between the valve and a urethral wall. The external seal member may include a foamed material. The external seal member may include an antimicrobial and/or antifungal agent. Only the magnetic valve member and ferromagnetic ring may not be elastically deformable. The valve seat may at least partially receive the magnetic valve element. At least 10% of the magnetic valve element may be received by the valve seat. Less than 50% of the magnetic valve element may be received by the valve seat. The valve main body may be tapered towards the outlet port.

Another aspect provides a valve system including a valve as above, a catheter coupled to the inlet port of the valve, and a retention mechanism coupled to the catheter, wherein the retention mechanism is configured for deployment in a bladder.

The valve system may further include: an extendible element extending through the valve and catheter, and secured to at least part of the retention mechanism; and a retaining clip configured to engage the extendible element and inhibit the movement thereof through the catheter. The valve system may further include an external retention mechanism configured to inhibit migration of the valve system into the bladder. The external retention mechanism may include an adjustable retention ring or a foam pad. The valve system may further include one or more external seal members located adjacent the external retention mechanism. A second catheter may couple the external retention mechanism to the valve. The valve system may further include one or more tethers for use in extraction of the valve system.

Another aspect provides a valve system as above in combination with an insertion tool for use in implantation of the valve system, wherein the insertion tool includes a sleeve member configured to receive at least part of the valve system.

The sleeve member may be more rigid than the valve system. The valve system may further include an inflatable external seal member. The valve system may further include one or more external seal members configured for attachment to the catheter and/or valve after implantation, each such external seal member comprising an annular member configured to be slide along a length of the catheter and/or valve. The valve system may further include at least one internal seal member configured to be received by at least part of the catheter such that a rib-like protrusion is formed in an outer surface of the catheter. The valve system may further include a sleeve member extending along at least part of a length of the catheter and/or valve, wherein the sleeve member defines one or more holes therethrough and/or includes one or more area formed of a different material to another part of the sleeve member and/or has a different texture to another part of the sleeve member.

Another aspect provides a valve system in combination with an insertion and/or extraction tool for use in implantation and/or extraction of the valve system, wherein the insertion and/or extraction tool includes an engagement member to engage with the valve system and the engagement member includes at least one of: a threaded engagement member, a magnetic engagement member, a hook, a hinged hook, a deployable retention member, and a loop configured to fit around at least part of the valve system.

The valve system may be for use with a human male or human female patient. The valve system may be configured for sterile storage with the retention mechanism in an undeployed configuration.

Another aspect provides an intraurethral valve system, including: an intraurethral valve for the selective flow of fluid therethrough; a retention mechanism to secure the valve system in place; a catheter coupled between the intraurethral valve and the retention mechanism to allow the passage of fluid to the intraurethral valve; an extendible element coupled to the retention mechanism and passing through at least part of the catheter for using an insertion of the intraurethral valve system; and a retaining clip configured to engage the extendible element and inhibit the movement thereof through the catheter.

The retaining clip may include a first and a second elongate member coupled by a hinge and configured to move about the hinge with respect to each other between open and closed configurations, and an engagement mechanism to hold the engage the first and second elongate members in the closed configuration. The retaining clip may include two elongate members coupled in a U-shaped configuration and defining a slit therebetween for receipt of the extendible member. A length of the retaining clip may be greater than a width of a lumen of the catheter. The valve system may be for use with a human male or human female patient. The valve system may be configured for sterile storage with the retention mechanism in an undeployed configuration.

Embodiments are described, by way of example only, with reference to the accompanying drawings, in which:
Figure 1 shows an extracted figure from WO00/02499;
Figure 2 shows an extracted figure from US6066088;
Figure 3a shows an embodiment of a valve;
Figure 3b shows another embodiment of a valve;
Figure 4a shows a valve of an embodiment in a closed configuration;
Figure 4b shows a valve of an embodiment in an open pass-through configuration;
Figure 4c shows a valve of an embodiment in an open configuration;
Figure 5 shows an end view of a ferromagnetic ring;
Figure 6 shows a side view of a ferromagnetic ring;
Figures 7a-7d show different external seal member locations and configurations;
Figure 8 shows a retaining clip of some embodiments coupled to part of a valve system;
Figure 9a shows a retaining clip in an open configuration
Figure 9b shows a retaining clip in a partially closed configuration;
Figure 9c shows a retaining clip in a closed configuration;

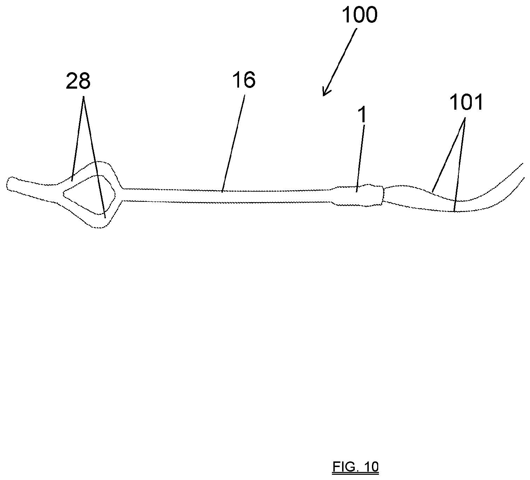

Figure 10 shows an embodiment with tethers;
Figure 11 shows a valve system (without the valve) in combination in an insertion tool of some embodiments;
Figures 12a-12c show a valve system (with different valve locations) in combination in an insertion tool of some embodiments;
Figure 13 shows a magnetic member and a valve, schematically;
Figure 14 shows an inflatable member along a valve system;
Figure 15 shows an example external seal member;
Figure 16 shows an example set of external seal members;
Figure 17 shows an example of the use of an internal member to create an external rib-like protrusion;
Figure 18 shows a sleeve member of some embodiments;
Figure 19 shows a valve with a tapered end according to some embodiments;
Figures 20a-20l show example extraction and/or insertion tools of some embodiments;
Figures 21a-21d show a valve system of some embodiments;
Figures 22a-22b show different views of the valve system of some embodiments (such as the embodiment of figures 21a-21d);
Figures 23a-23b show a valve system with a partial cut-away to depict the open and closed configurations of the valves;
Figures 24a-24d show an embodiment of a valve;
Figures 25a-25b show different views of the embodiment of figures 24a-24b;
Figure 26 shows details of parts of a valve of some embodiments;
Figures 27a-27d show an embodiment of a valve;
Figures 28a-28b show different views of the embodiment of figures 27a-27b;
Figures 29a-29d show a valve system of an embodiment, with figure 29d showing details of the valve of that, and some other, embodiments;
Figures 30a-30d show a valve system of an embodiment, with figures 30a and 30d showing the indicated details of the valve of that, and some other, embodiments;
Figures 31a-31f show a valve seat member of some embodiments; and
Figures 32a-32b show parts of a valve of some embodiments.

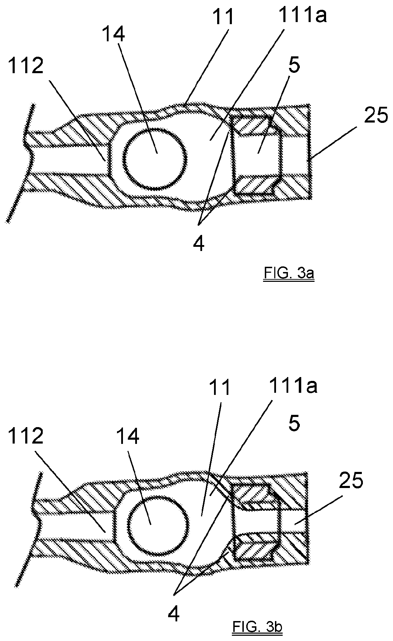

Embodiments may include a valve 1 - see figures 3-6. The valve 1 may be a magnetically actuated valve 1 for intraurethral placement. Therefore, the valve 1 may be an intraurethral magnetic valve 1.

With reference to figures 3a and 3b by way of example, the valve 1 may include a valve main body 11 defining an internal valve volume 111 in which is positioned a spherical magnetic valve element 14 (hereinafter "the valve element 14"). The internal valve volume 111 has a length along a longitudinal axis of the valve main body 11. The longitudinal axis of the valve main body 11 extends from an inlet port 112 of the valve 1 to an outlet port 25 of the valve 1. The internal valve volume 111 has a width along an axis perpendicular to the longitudinal axis of the valve main body 11.

In some embodiments, the internal valve volume 111 may be generally cylindrical in shape and the width thereof may be a diameter, for example. In some embodiments, the internal valve volume 111 may generally narrow from the end thereof adjacent the outlet port 25 towards the end thereof adjacent the inlet port 112. In some embodiments, there may be a bulbous portion 111a of the internal volume 111, which may be towards the outlet port 25. The bulbous portion 111a may be a part of the internal volume 111 which is wider than at least part of the rest of the internal volume 111.

The inlet port 112 and the outlet port 25 may both have a width (e.g. a diameter) which is less than that of the internal valve volume 111.

The internal valve volume 111 is sized to receive the valve element 14, for example, as depicted in figure 3. The internal valve volume 111 may be sized to permit movement of the valve element 14 between a closed configuration and an open configuration - as described herein.

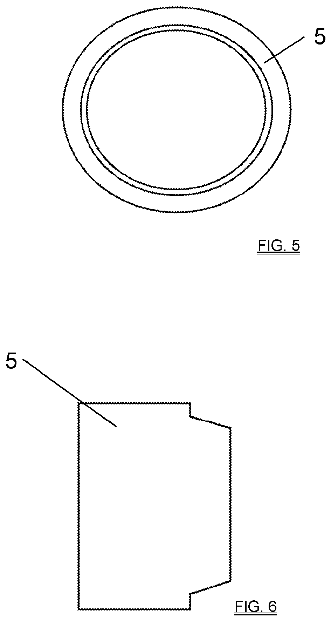

Towards the outlet 25, there is a ferromagnetic ring 5. The ferromagnetic ring 5 defines a passage through which fluid may pass. The ferromagnetic ring 5 is depicted in the valve 1 in figures 3a and 3b, as well as separately in figures 5 and 6, for example.

The ferromagnetic ring 5 may be a continuous ring of ferromagnetic material - such as depicted in figures 5 and 6, in which figure 5 shows an end view of the ferromagnetic ring 5 and figure 6 shows a side view. Accordingly, the ferromagnetic ring 5 may be generally tubular in form. In some embodiments, the ferromagnetic ring 5 may be ring-like, in that a complete ring may not be formed by ferromagnetic material may form a ring-like structure formed of a plurality of ferromagnetic material segments.

The ferromagnetic ring 5 may have a first end which is, when in the valve 1, towards the internal volume 111 and a second end which is, when in the valve 1, away from the internal volume 111 (relative to the first end). The first and second ends of the ferromagnetic ring 5 may oppose each other across a length of the ferromagnetic ring 5 (a length which may be parallel to the longitudinal axis of the valve 1 when the ferromagnetic ring 5 is located in the valve 1).

In some embodiments, the first end of the ferromagnetic ring 5 may have a generally flat and/or smooth end surface (which may, as will be appreciated, be an annular surface).

The ferromagnetic ring 5 may have a middle portion extending from the first end towards the second end which has a generally constant external radius and which may additionally or alternatively have a generally constant internal radius.

In some embodiments, the second end of the ferromagnetic ring 5 may include a stepped portion which may include portions of decreasing external and/or internal radius towards an end surface of the second end. In some embodiments, this stepped portion extends from the middle portion to the end surface of the second end of the ferromagnetic ring 5.

In some embodiments, the ferromagnetic ring 5 has a substantially uniform internal diameter, and/or external diameter, along its entire length.

The ferromagnetic ring 5 may be coupled to the valve main body 11 and this may be achieved in several different manners.

In some embodiments, the valve main body 11 is formed from an elastomer material and may be formed from only the elastomer material. This elastomer material may be selected for its biocompatibility and/or the ability to impregnate the material with antimicrobial and/or antifungal agent. The ferromagnetic ring 5 may be formed of a ferrous metal or ferrous metal alloy. Therefore, the valve body 11 may be formed from a relatively flexible material compared to the ferromagnetic ring 5. In some embodiments, application of a force to the valve main body 11 may cause the valve main body 11 to deform elastically and for the internal valve volume width (in the direction of the force) to be reduced. In other words, the main body 11 may be resilient to crushing forces to which it may be exposed.

In some embodiments, the ferromagnetic ring 5 may be embedded in a least part of the valve main body 11 with the valve main body 11 at least partially formed around the ferromagnetic ring 5. In such embodiments, the formation of the valve main body 11 (or at least the part in which the ferromagnetic ring 5 is embedded) may be a moulding process around the ferromagnetic ring 5 - which may, therefore, be placed inside a mould before elastomer material for the valve main body 11 (or part thereof) delivered to the mould (e.g. injected into the mould).

In some embodiments, the ferromagnetic ring 5 may be secured by an interference fit within the valve main body 11. In such embodiments, the valve main body 11 may be formed separately from the ferromagnetic ring 5, with a recess defined by the valve main body 11 for receipt of the ferromagnetic ring 5. The ferromagnetic ring 5 may then be inserted into the recess and held in place by, for example, an interference fit between the valve main body 11 and one or more surfaces of the ferromagnetic ring 5. In some embodiments, a plug of material (which may be elastomer material such as that which may be used for the valve main body 11) may be used to secure, or help to secure the ferromagnetic ring 5 in place. This plug may be located towards the outlet port 25 of the valve 1 and, indeed, may define the outlet port 25 - the plug may be annular in form, for example, with the outlet port 25 defined by the plug.

In some embodiments, the ferromagnetic ring 5 may be fitted to a spigot member of the valve main body 11. The spigot member may define a passage therethrough and the ferromagnetic ring 5 may slide over the spigot member into position. A plug of material may then fit over the ferromagnetic ring 5. The plug of material may be generally annular in form.

In some embodiments, adhesive may be used to secure, or help to secure, the ferromagnetic ring 5 to the valve main body 11. In some embodiments, it will be understood that the spigot member may be part of the valve main body 11 and, likewise, the plug of material may form part of the valve main body 11.

In some embodiments, the a valve main body 11 may be of a tubular form with one open end and with the ferromagnetic ring 5 located at an opposing end (the opposing end forming the outlet 25 which is generally smaller in width than the open end). The open end may be configured to be fitted to another portion of the valve main body 11 and/or an end of a catheter 16 - the other portion defining the inlet port 112 or an outlet of the catheter 16 forming the inlet port for the valve 1. Therefore, the valve element 14 may be located within the valve main body 11 through the open end thereof prior to coupling to the other portion of the main body 11 and/or the end of the catheter 16 (the other portion of the main body 11 may be coupleable to the catheter 16). The catheter 16 may be integrally formed with at least part of the valve 1 (such as the main body 11), or may be adhered thereto, or may be connected using a cohesive process (such as insert molding).

In some embodiments in which the valve main body 11 is not moulded around the ferromagnetic ring 5 during manufacture, the valve element 14 may be inserted through the outlet port 25 (or the end of the valve main body 11 at which the outlet port 25 will be located) prior to insertion of the ferromagnetic ring 5 (and, for example, a plug). The elastomer material of the valve main body 11 may be configured to elastically deform to allow the insertion of the valve element 14 in this manner, for example.

The valve 1 includes a valve seat 4 which may be a non-ferromagnetic valve seat 4, for example. The valve seat 4 could be formed in a number of different manners according to embodiments. For example, the valve seat 4 may, in some embodiments, be formed by the material which forms the valve main body 11 in which the ferromagnetic ring 5 is located.

The portion of the valve seat 4 which is configured to abut the valve element 14 may have a radius which matches or substantially matches an external radius of the valve element 14. In some embodiments, the portion of the valve seat 4 which is configured to abut the valve element 14 may be configured to deform elastically on contact with the valve element 14. Such arrangements may be configured to improve the seal between the valve seat 4 and the valve element 14.

In some embodiments, the valve seat 4 may include a coating of a valve seat material, may be impregnated with a valve seat material, or may otherwise include an additive in the form of the valve seat material. This valve seat material may include an antimicrobial and/or antifungal agent, and/or a ferromagnetic material (which may be in powdered form), and/or a hydrophilic or hydrophobic material. The use of a valve seat material may, in this manner, seek to improve a seal between the valve seat 4 and the valve element 14 and/or reduce a build-up of microbial material on the valve seat 4.

In some embodiments, a separate ferromagnetic ring 5 may not be used. For example, the valve seat 4 (and/or valve seat member 41) may be formed from an elastomer (e.g. silicone) including a filler material such as miniature steel or iron spheres or powder. In some embodiments the ferromagnetic ring 5 is provided as a separate piece to the valve seat 4 (and/or valve seat member 41) but is, itself, formed from an elastomer (e.g. silicone) including a filler material such as miniature steel or iron spheres or powder.

In some embodiments, therefore, the end surface at the first end of the ferromagnetic ring 5 may be covered by the material which forms the valve main body 11. Two examples of this are depicted in figures 3a and 3b.

In some embodiments, with reference to figure 3a for example, the material of the valve main body 11 covers the end surface at the first end of the ferromagnetic ring 5; however, in this and some other embodiments, at least part of the passage through the valve 1 may be defined by the ferromagnetic ring 5 such that fluid passing therethrough may contact the ferromagnetic ring 5, for example (or at least part thereof). The valve seat 4 in these embodiments may be formed by the material covering the end surface at the first end of the ferromagnetic ring 5.

In some embodiments, with reference to figure 3b for example, the material of the valve main body 11 covers the end surface at the first end of the ferromagnetic ring 5 (much as in the embodiment of figure 3a) but the rest of the ferromagnetic ring 5 is also substantially covered by the material of the valve main body 11 - such that fluid passing therethrough does not contact the ferromagnetic ring 5 (or at least does not contact a substantial part thereof). The valve seat 4 in these embodiments may be formed by the material covering the end surface at the first end of the ferromagnetic ring 5.

In some embodiments, a separate valve seat member 41 is provided to form the valve seat 4. This separate valve seat member 41 may be located adjacent the end surface at the first end of the ferromagnetic ring 5 and may be secured to the ferromagnetic ring 5 and/or at least partially embedded in the material of the valve main body 11. The valve seat member 41 may be formed of a stiffer elastomer material than the valve main body 11, for example, or otherwise may have one or more properties which enhance the seal between the valve element 14 and the valve seat 4 (compared to using the material of the valve main body 11 for the valve seat 4). In some embodiments, the valve seat member 41 may improve consistency in the formation of the valve seat 4 (again, compared to using the material of the valve main body 11 for the valve seat 4). The valve seat member 14 may be formed from a different durometer silicone than the valve main body 11 (which may also be formed of silicone).

The valve 1 is actuatable between open and closed configurations. In the open configuration, the valve element 14 is spaced apart from the valve seat 4 such that fluid may flow around the valve element 14 and through the outlet 25. In the closed configuration, the valve element 14 is seated against the valve seat 4 such that the outlet port 25 is substantially sealed from fluid communication with the inlet port 112.

As will be appreciated, with the valve element 14 may be biased toward the closed configuration by virtue of magnetic attraction to the ferromagnetic ring 5. In addition, with the valve 1 located in a urethra as an intraurethral valve and oriented with respect to the normal flow of fluid therethrough, the inlet port 112 may be upstream of the outlet port 25 with fluid, such that the flow of fluid through the valve 1 may also assist in biasing the valve member 14 towards the closed configuration.

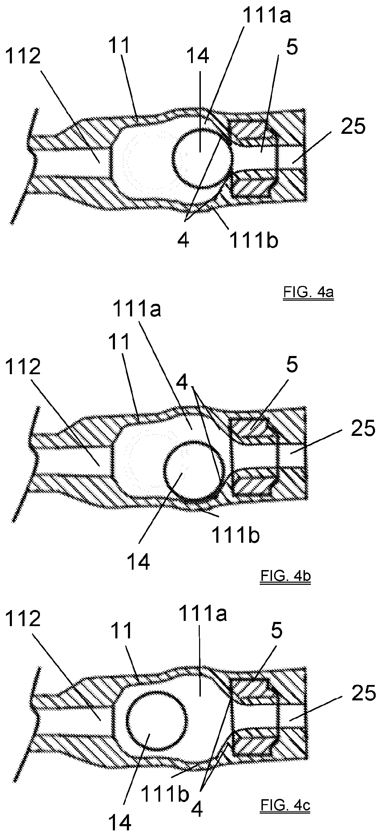

An embodiment (the embodiment of figure 3b) is shown in figure 4a in the closed configuration and in figure 4c in the open configuration, by way of example only. Figure 4b shows a variation of the open configuration in which a path is provided through the valve 1 for allowing an extendible element 50 to pass therethrough for insertion of the valve 1 into the patient - for example, using the implanting system of WO2008/067557 or a variation thereof.

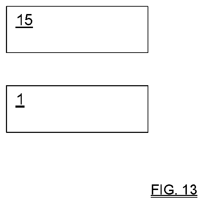

Actuation of the valve 1 to the open configuration may be achieved through the use of a magnetic member 15 (see figure 13). The magnetic member 15 may be positioned external to the patient to which the valve 1 is fitted and moved into relative close proximity to the valve 1 to achieve actuation from the closed to the open configuration (through expose of the valve 1 to the magnetic field of the magnetic member 15).

Actuation to the closed configuration may be achieved by magnetic attraction between the valve element 14 and the ferromagnetic ring 5, and may be aided by one or both of gravity and the flow of fluid through the valve 1, for example.

The valve 1 described herein may be fitted to a male patient or a female patient as an intraurethral magnetic valve 1. With reference to figure 1, the valve 1 described herein can take the place of the valve (1) shown in figure 1 and described in the document from which that figure was taken - see above for the document details.

As will be appreciated, the valve 1 is substantially formed of a relatively flexible material - e.g. an elastomer material. In some embodiments, only the valve element 14 and the ferromagnetic ring 5 are not formed from such a material (and in some embodiments, the valve seat member 41 may be formed of a different material).

Providing such a valve 1 may reduce discomfort in some circumstances due to reduced bulk of the design compared to some other valves and/or due to the majority of the valve 1 being formed from a relatively flexible material (relatively flexible compared to the valve element 14 and ferromagnetic ring 5, for example). The reduced bulk and/or flexible body of the valve 5 may also aid insertion into the urethra.

The valve main body 11 may, as described above be formed of a relatively flexible material (such as an elastomer material). An outer surface of the valve main body 11 may, therefore, be formed of such a material. This may aid in the formation of a seal between the valve 1 and a wall of the urethra, for example. This may help to reduce the leakage of fluid around the valve 1.

An external width (which may be diameter) of the valve 1 may be substantially the same as or marginally greater than a width of the part of the urethra at which the valve 1 is intended to be located - see below regarding location of the valve 1. Multiple different widths of valve 1 may be provided for different urethra part widths, for example, so that a relatively tight fit can be achieved. At least part of the outer surface of the valve 1 - e.g. part of the valve main body 11 - may be configured to deform elastically on insertion into a urethra. At least part of the outer surface of the valve 1 - e.g. part of the valve main body 11 - may be configured to be elastically deformed with the valve 1 at its intended location. This at least a part, may be intended to remain elastically deformed with the valve 1 at its intended location - although the precise form of the deformation may vary over time.

In some embodiments, the valve 1 includes one or more external features which are configured to increase the engagement between the valve 1 and the wall of the urethra. These one or more external features may be formed in the outer part of the valve main body 11 and may be features which are configured to deform elastically (e.g. as described above).

The one or more external features may include one or more ribs or other protrusions which may extend generally radially outwardly from the valve main body 11. In some embodiments, the or each external feature may include at least one circumferential rib which may extend around all or a substantially part of a circumference of the valve main body 11.

In some embodiments, at least one of the one or more external features has a corresponding internal feature within the internal valve volume 111. For example, the bulbous portion 111a may have a corresponding external rib 111b which is at least partially defined by the valve main body 11.

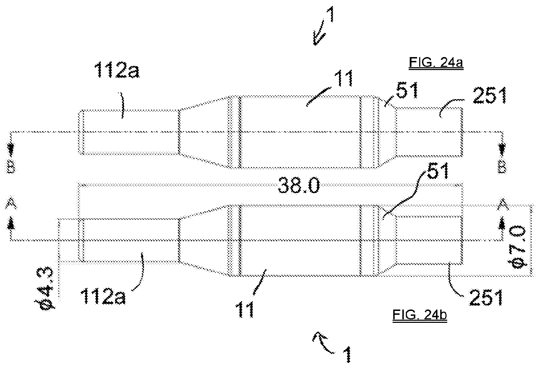

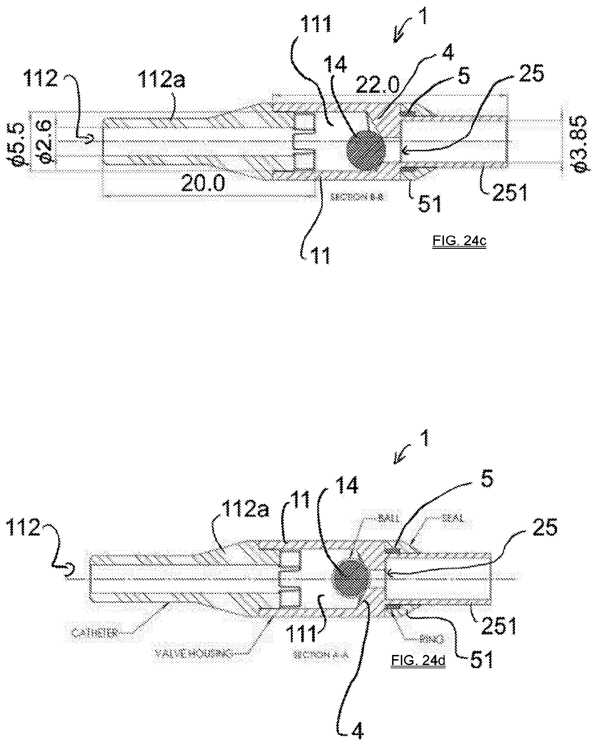

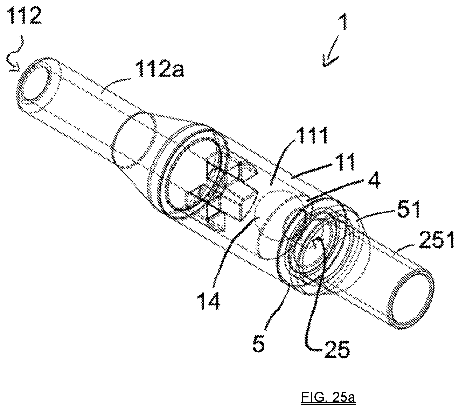

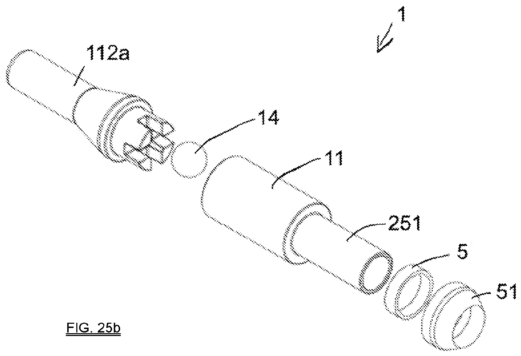

Further embodiments of the valve 1 are depicted in figures 24a-24d, 25a-25b, 27a-27d, and 28a-28b.

With reference to figures 24a-24d, the valve 1 may include an inlet end and an outlet end (the outlet port 25 being generally located at outlet end and the inlet port 112 being generally located at the inlet end).

The valve main body 11 may include a tubular body in which the valve seat 4 is located and with which the valve seat 4 may be integrally formed. The outlet port 25 may be offset with respect to a central longitudinal axis of the valve 1 - such offset valve seats 4 are described elsewhere herein.

The tubular body of the valve main body 11 may, therefore, form a cup-like member with a aperture (the outlet port 25) there through and an open opposing end.

The open end of the tubular main body 11 (e.g. the tubular body towards the inlet end) may be configured to receive a tubular plug 112a. The tubular plug 112a may be configured to be at least partially received by the tubular main body 11 to define the internal valve volume 111. In manufacture, the spherical magnetic valve element 14 may be positioned with the tubular main body 11 and the tubular plug 112a then secured thereto. An adhesive may be used to help to secure the tubular main body 11 and tubular plug 112a. The tubular plug 112a may define the inlet port 112 and may be integrally formed with the catheter 16, for example (or attachable thereto). An end of the tubular plug 112a may include a plurality of teeth. The teeth may extend in a longitudinal direction and may be spaced apart circumferentially. The provision of such teeth may provide increased radial flexibility of the end of the tubular plug 112a. The teeth may be received by the tubular main body 11.

An outer wall of the tubular plug 112a may have a diameter which tapers away from the part which is received (or receivable) by the tubular main body 11.

The outlet port 25 may be in fluid communication with an outlet tube 251 of the valve 1. The outlet tube 251 may have a smaller external diameter than the tubular main body 11 of the valve 1.

The ferromagnetic ring 5 may be configured to fit over at least part of the outlet tube 251. The ferromagnetic ring 5 may be configured to be located around at least part of the outlet tube 251 and/or the tubular main body 11 so as to be located relative to the valve seat 4.

An annular cover member 51 may be located over the ferromagnetic ring 5. This annular cover member 51 may be configured to pass over a portion of the outlet tube 251 and may be secured thereto, and/or to the ferromagnetic ring 5, and/or to the tubular main body 11. An adhesive may be used to secure the ferromagnetic ring 5 and/or the annular cover member 51 in place.

An outer diameter of the annular cover member 51 may taper away from the tubular main body 11.

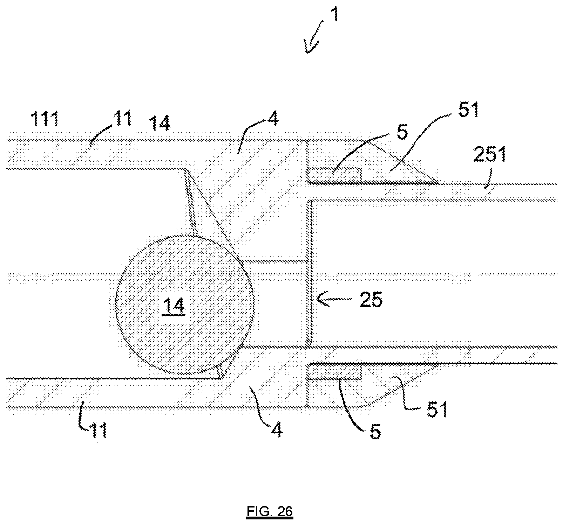

The arrangement of the spherical magnetic valve element 14 (in the closed configuration), with respect to an offset valve seat 4, and including the ferromagnetic ring 5 and the annular cover member 51 is depicted in figure 26, for example.

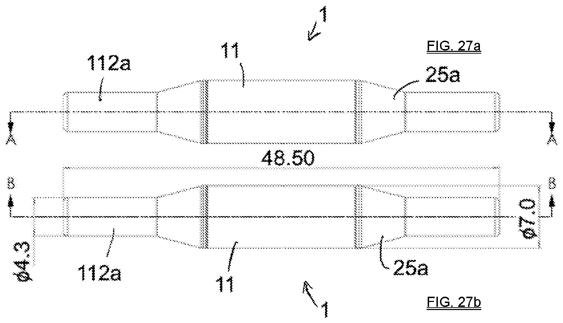

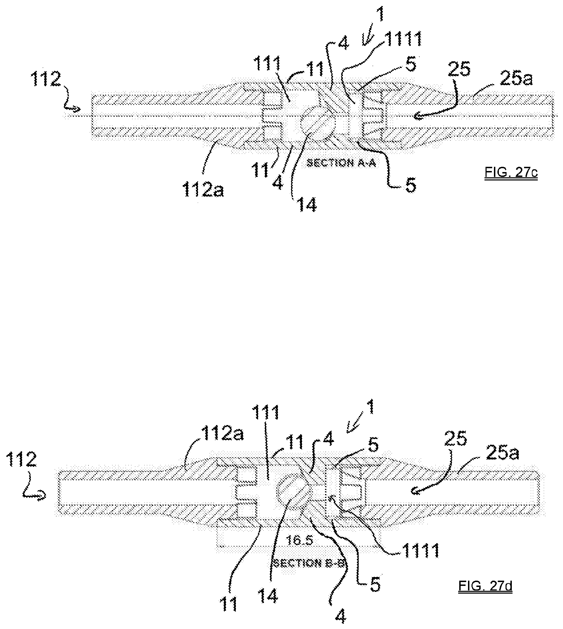

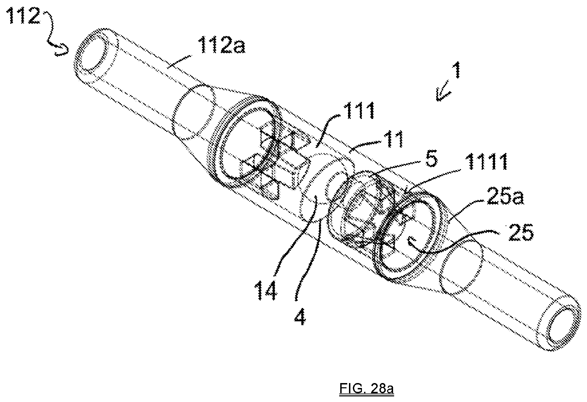

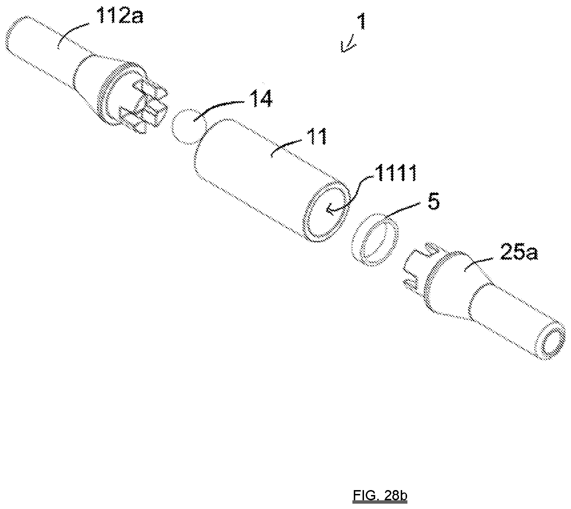

Another embodiment is depicted in figures 27a-27d, and figures 28a-28b. In this embodiment, the inlet end tubular plug 112a is generally of the same form as in the preceding embodiment. In this embodiment, and those like it, however, there is also an outlet port tubular plug 25a.

The tubular main body 11 may, therefore, be configured to receive at least part of the inlet and outlet tubular plugs 112a, 25a at either end thereof.

The inlet tubular plug may be at least partially received by the internal valve volume 111. In some embodiments, the tubular main body 11 may define an outlet volume 1111 which is configured to receive at least part of the outlet tubular plug 25a. The outlet volume 1111 may be further configured to receive the ferromagnetic ring 5 - which may adhered in place and/or which may be held in place by the outlet tubular plug 25a. An end of the outlet tubular plug 25a which is received by the outlet volume 1111 may include teeth in the same manner as the inlet tubular plug 112a as described herein. An external diameter of the outlet tubular plug 25a may taper away from the tubular main body 11.

The valve seat 4 may be integrally formed with and/or tubular main body 11 or may be secured inside the tubular main body 11.

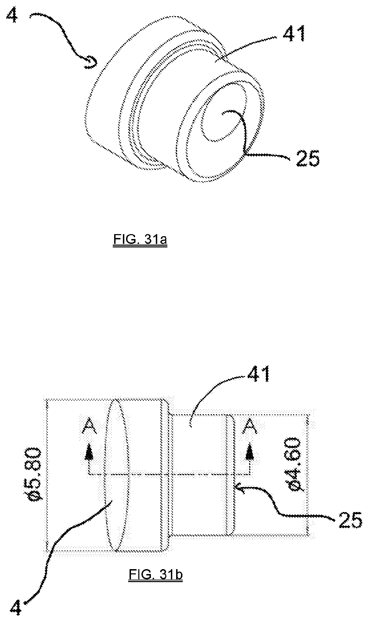

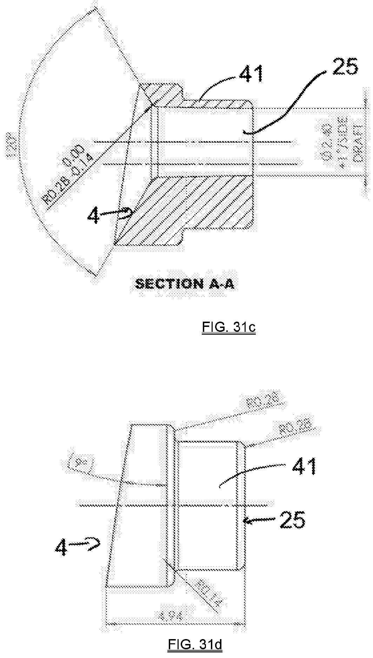

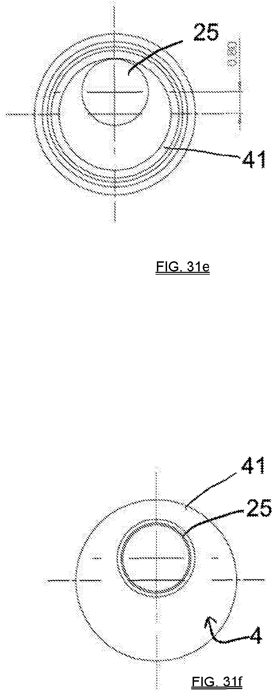

In some embodiments, the valve seat 4 may be part of a valve seat member 41 which may be secured to the tubular main body 11. Some embodiments, of the valve seat 4 described with reference to figures 31a-31f. The valve seat 4 may the same general form if integrally formed with the tubular main body 11, for example.

The valve seat 4 is an offset valve seat - e.g. with an outlet port 25 which is offset from a central longitudinal axis thereof (the central longitudinal axis being parallel to the normal direction of fluid flow through the valve seat member 41).

The valve seat 4 (which may be part of the valve seat member 41) may define a cup-like formation though the base of which the outlet port 25 may be provided. The valve seat 4 may, therefore, be configured to receive at least part of the spherical magnetic valve element 14.

The valve seat 4 may be shaped such that a portion of the valve element 14 is received by the valve seat 4 when the valve 1 is in the closed configuration. In some embodiments at least 10% of the valve element 14 is so received. In some embodiments at least 20% of the valve element 14 is so received. In some of these and other embodiments less than 50% of the valve element 14 is received in this manner by the valve seat 4.

The cup-like valve seat 4 of some embodiments may likewise be described as bowl-like.

As mentioned, the valve seat 4 may be formed of a resiliently deformable material (e.g. an elastomer) such that the valve element 14 may deform at least part of the valve seat 4 when in the closed configuration. This may allow for wider tolerances in relation to the site and shape of the valve seat 4 and/or valve element 14.

Likewise, the resiliently deformable valve seat 4 and/or the cup-like valve seat form may improve the seal (in the closed configuration) despite any build-up of material (i.e. encrustation) in the valve 1. The valve element 14 may also be coated in an antimicrobial and/or antifungal material and this is made more practical by one or both of these features (e.g. because there are wider tolerances).

Due to the offset form of the valve seat 4 in some embodiments, the valve seat 4 may include a circumferential inclined surface which is configured to contact the spherical magnetic valve element 14 and a radial depth of the valve seat 14 may be larger in one part compared to an opposing part. Example angles of inclination of the valve seat 14 are shown in figure 31c - by way of example only. In some embodiments, the indicated angles may be ± 10-20%.

The valve seat member 41 may have a first outer diameter in the region of the valve seat 4 which is larger than a second outer diameter of the valve seat member 41 in the region of the outlet port 25. This may enable, for example, the fitting of the valve seat member 41 to the tubular main body 11 and/or to an outlet tubular member (which may be in fluid communication with the outlet port 25). The reduced diameter (i.e. the second outer diameter) may be configured to permit the ferromagnetic ring 5 to pass to be secured to the valve seat member 41.

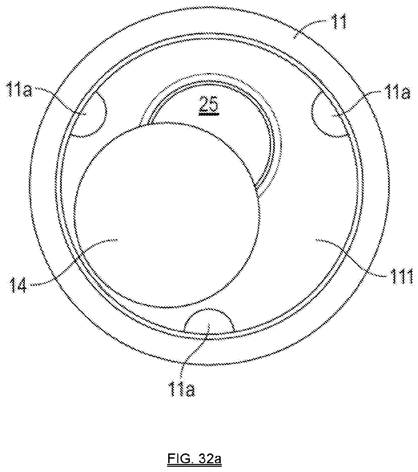

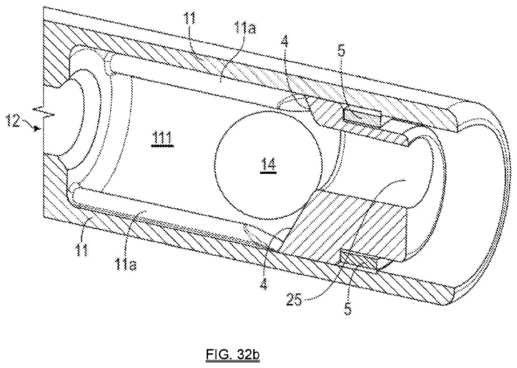

With reference to figures 32a and 32b, the tubular main body 11 may define one or more internal elongate ribs 11a, each of which may extend into the internal valve volume 111. The or each internal elongate rib 11a may be generally aligned with a longitudinal axis of the tubular main body 11. In some embodiments, there are a plurality of such elongate 11a ribs spaced around in internal circumference of the tubular main body 11. The or each elongate rib 11a may extend from the inlet end towards the outlet end of the tubular main body 11. The or each internal elongate rib 11a may help to guide the valve element 14, and/or may reduce the risk of the valve element 14 adhering to the main body 11.

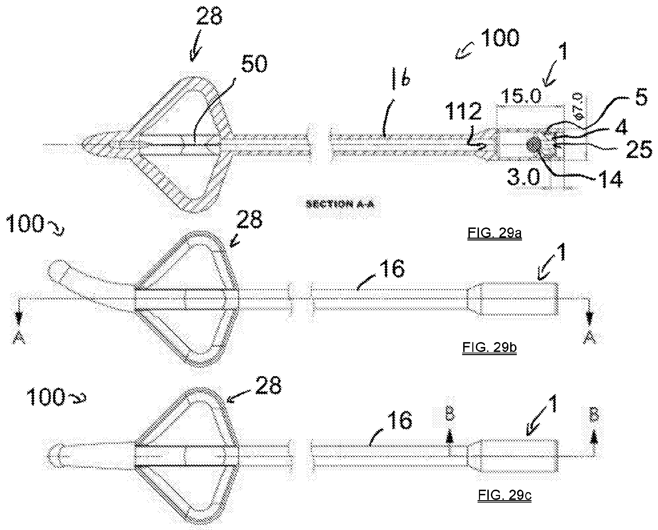

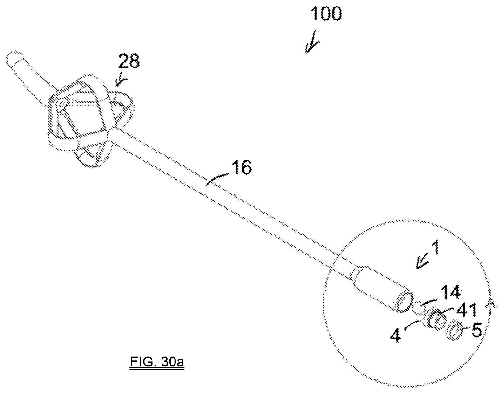

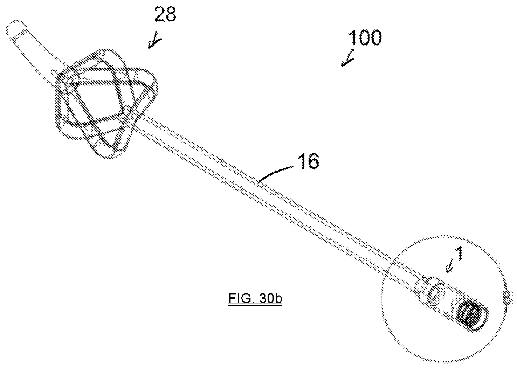

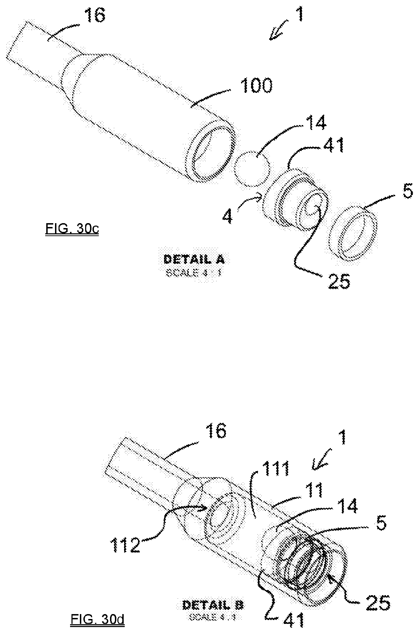

In figures 29a-30d there is depicted an embodiment, in various views, which include the aforementioned valve seat member 41 as part of a valve 1 in a similar manner to those described elsewhere herein.

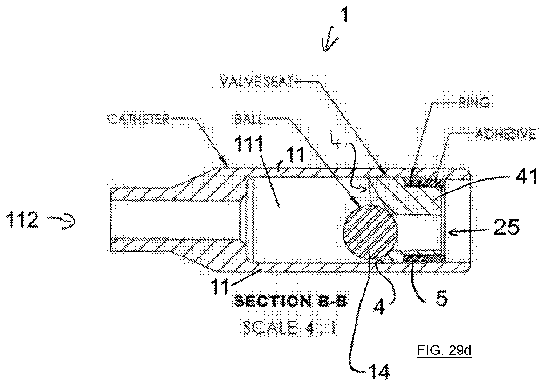

With reference to figure 29d as an example of the valve 1, the valve seat member 41 may be configured to close or substantially close the outlet end of the tubular main body 11 (taking into account that the valve seat member 41 defines the outlet port 25 of course). The valve seat member 41 may, therefore, form an outlet end plug. The ferromagnetic ring 5 may be provided around at least a portion of the valve seat member 41 and may be received within the tubular main body 11. Adhesive may be used to secure the valve seat member 41 and/or the ferromagnetic ring 5 in the tubular main body 11.

The valve seat member 41 and the ferromagnetic ring 5 may be correspondingly sized and shaped to permit the ferromagnetic ring 5 to fit around at least part of the valve seat member 41. In some embodiments, the valve seat member 41 and ferromagnetic ring 5 are keyed such that the rotational position between the two parts may be controller (.e.g. allowing a single or a discrete plurality of relative rotational positions with respect to each other when fitted).

In some embodiments, the valve 1 may include an external seal member 113. Examples of external seal members 113 are shown schematically in figured 7a-7d.

In particular, the external seal member 113 may be located external to the valve main body 11 and may extend around a portion of an external surface thereof. In some embodiments, such as those depicted, the seal member 113 is a generally annular member which extends around substantially an entire circumference of the valve main body 11. The external seal member 113 may be formed of a different material to the valve main body 11 and may be a more flexible material, for example. In some embodiments, the external seal member 113 may be a foamed material, such as a foamed elastomer material. The external seal member 113 (which may be a foamed material) may be impregnated or coated with an antibacterial material and/or may be formed of an antibacterial material. Additionally, or alternatively, the external seal member 113 (which may be a foamed material) may be impregnated or coated with a hydrophilic material and/or may be formed of an hydrophilic material. Accordingly, at least part of the length of the catheter 16 may have a hydrophilic coating (whether provided by the external seal member 113 or otherwise).

The external seal member 113 may be configured to provide an improved seal between the valve and the urethra to reduce the likelihood of leaks around the valve 1. In some embodiments, the seal member 113 may help to retain the valve 1 is place within the urethra.

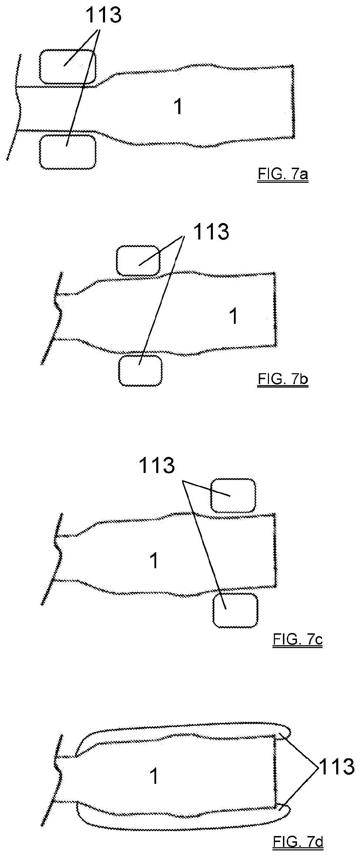

In the example in figure 7a, the external seal member 113 is located at the end of the valve 1 towards the inlet port 112 and may be, in fact, located around a part of the catheter 16 coupled to the valve 1.

In the example in figure 7b, the external seal member 113 is located around a portion of the valve main body 11 towards the end of the valve 1 with the inlet port 112 (e.g. between the external rib 111b if provided and the inlet port 112).

In the example in figure 7c, the external seal member 113 is located around a portion of the valve main body 11 towards the end of the valve 1 with the outlet port 25 (e.g. between the external rib 111b if provided and the outlet port 25).

In the example in figure 7d, the external seal member 113 is in the form of a coating which covers substantially all of the valve main body 11 from end with the inlet port 112 to the end with the outlet port 25.

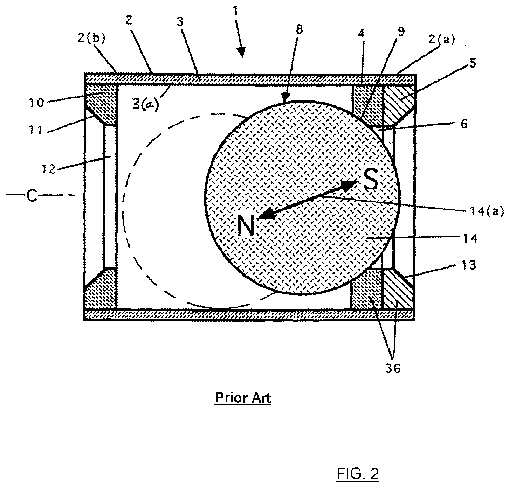

In some embodiments, there may be multiple seal members 113 which may be any combination of the seal members 113 described herein and shown in figured 7a-7d. So, for example, there may be two seal external seal members 113 comprising the member 113 of figure 1 and the member 113 of figure 2, or the member 113 of figure 2 and the member 113 of figure 3. In some embodiments, for example, there may be three seal external seal members 113 comprising the member 113 of figure 1 and the member 113 of figure 2 and the member 113 of figure 3. The seal member 113 of figure 7b may be provided with one or more ribbed portions to provide corresponding seal member portions to the seal members 113 shown in one or more of figures 7a-7c.

As mentioned above, the valve 1 may be coupled to catheter 16. The catheter 16 provides and defines a fluid flow path to the valve 1 and, in particular, to the inlet port 112 of the valve 1. The valve 1 may be an intraurethral valve 1 and the catheter 16 may, accordingly, be coupled to a retention mechanism which is configured to secure or help to secure the valve 1 and catheter 16 in the desired location.

The retention mechanism could take a number of different forms and is configured to be passed through a urethra to a desired location before being deployed. On deployment, the retention mechanism helps to hold the catheter 16 and/or valve 1 in position. In some instances, deployment generally means the expansion or extension of at least part of the retention mechanism.

In some embodiments, the retention mechanism may comprise a malecot - such as is shown in figure 1. The retention mechanism may include a plurality of arms or loops or rings 28 which act, when deployed, to restrict movement of the catheter 16 and/or valve 1 which are coupled to the retention mechanism. The arms or loops or rings 28 are, therefore, moveable from an undeployed or retracted configuration to a deployed or extended configuration. Herein the arms or loops or rings 28 are examples of deployable members 28 of the retention mechanism and this term will be used hereinafter.

In some embodiments, therefore, there is provided a valve system 100 which includes: the valve 1, the catheter 16, and the retention mechanism.

The length of the catheter 16 from the valve 1 to the retention mechanism may be selected based on the desired placement of the valve 1 - as described herein, for example.

The retention mechanism may, as described above, include a plurality of deployable members 28. These deployable members 28 may extend radially outwardly with respect to the catheter 16 such that they define a part of the system 100 which is wider than the catheter 16 when deployed (but which may be retracted when in the undeployed configuration to be substantially the same width as the catheter 16, for example). The deployable members 28 may define spaces therebetween. These spaces may provide fluid communication between a volume surrounding the retention mechanism and a lumen 21 defined by the catheter 16. This volume, in the case of an intraurethral system, may be the bladder 29.

Accordingly, much as in figure 1, the retention mechanism may locate in the bladder neck 22 with the catheter 16 extending down (in terms of normal fluid flow direction) the urethra.

As mentioned herein above, the valve 1 of some embodiments may be inserted or otherwise implanted using the systems disclosed in, for example, WO00/02499 and/or WO2011/032150.

Generally, in summary reference to the teachings of documents such as WO00/02499 and/or WO2011/032150, insertion of the valve system 100 may include the removable connection of the valve system 100 to an insertion tool 200. The extendible element 50 mentioned above extends from the tool 200, through the outlet port 25 of the valve 1, through the internal valve volume 111 - e.g. with the valve 1 in the configuration depicted in figure 4b which will hereinafter be referred to as the open pass-through configuration - through the catheter 16 and to the retention mechanism. An end of the extendible element 50 (i.e. a proximal end) is removably secured to the retention mechanism.

The extendible element 50 may be a wire or the like and is configured to be pushed, using the insertion tool 200 for example, towards the retention mechanism so as to stiffen the valve system 100 for insertion. This action also places the retention mechanism in its undeployed (or retracted) configuration to enable the more ready passing thereof through the urethra.

Once the retention mechanism is located in the desired position - e.g. in the bladder - then the extendible element 50 may be released and retracted from the valve system 100, deploying the retention mechanism, and the insertion tool and extendible element 50 may be removed.

As can be seen from both WO00/02499 and WO2011/032150 the release and retraction of the extendible element 50 may require the use of a locking wire or magnetic elements in order to allow the extendible element 50 to remain captured by the retention mechanism until it is desired to release and extract the extendible element 50.

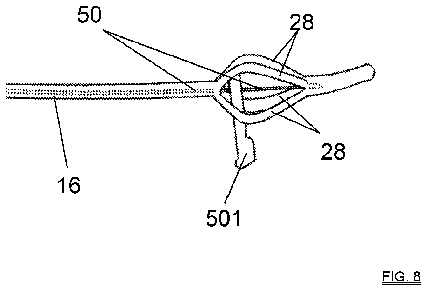

An embodiment, provides a retaining clip 501 for the extendible element 50 - see figure 8, for example.

The retaining clip 501 may be an elongate clip which is configured to be selectively clipped around the extendible element 50 such that a part of the retaining clip 501 also extends beyond the confines of the retention mechanism and, in this case, beyond the deployable members 28. The retaining clip 501, when secured to the extendible element 50 (which may be in the form of a wire or thin tube) may be configure to resist movement along a length of the extendible element 50 and may, therefore, be substantially prevented from such movement. The retaining clip 501 may, therefore, inhibit the movement of the extendible element 50 further into the catheter 16 due to abutment of the clip with at least part to the retention mechanism (e.g. one or more of the deployable members 28 or a part to which the deployable members 28 are attached) or the end of the catheter 16 itself.

The retaining clip 501 permits the valve system 100 to be stored for use with the extendible element 50 attached to the retention mechanism (or otherwise to a proximal end of the valve system 100). The valve system 100 may also be stored for use with the insertion tool 200. The retaining clip 501 may reduce the risk of the extendible element 50 being accidentally or erroneously removed from attachment to the proximal end of the valve system 100 (e.g. to the retention mechanism). Prior to use (i.e. insertion) of the valve system 100, the retaining clip 501 would be removed.

The retaining clip 501 could take a number of different forms.

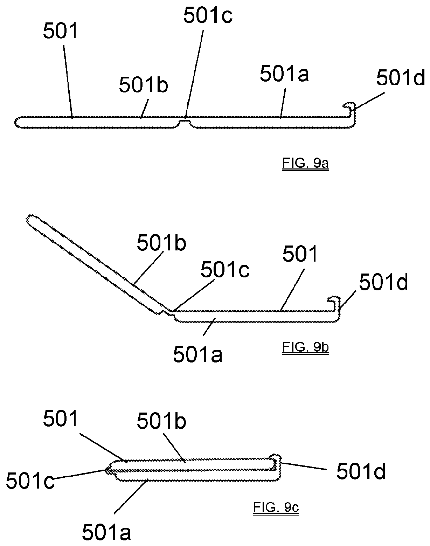

With reference to figures 8 and 9a by way of example only, the retaining clip 501 may include first and second hinged elongate members 501a, 501b. The first and second elongate members 501a,501b may be coupled at one end to each other by a hinge 501c such that the two elongate members 501a,501b are moveable with respect to each other about the hinge (see figure 9b).

The first and second elongate members 501a,501b may be formed from a plastics material and the hinge 501c may likewise be formed of a plastics material (but which may be thinner than the material of the first and second elongate members 501a,501b).

Remote from the hinge 501c, the first and second elongate members 501a,501b may be configured to engage and, to for this purpose, the retaining clip 501 may include an engagement mechanism 501d. The engagement mechanism 501d may include a part of one of the first and second elongate members 501a,501b which is configured to clasp around a part of the other of the first and second members 501a,501b. One or both of these parts may be configured to deform elastically as the retaining clip 501 moves from an open to a closed configuration such that the engagement mechanism holds the two elongate members together 501a,501b and substantially prevents movement of one elongate member 501a,501b with respect to the other 501a,501b, about the hinge 501c (see figure 9c).

The engagement mechanism may be selectively and manually releasable to allow the retaining clip 501 to adopt the open configuration by movement of one or both of the engaging parts out of engagement and rotation of one elongate member 501a,501b with respect to the other 501,501b about the hinge 501c. With the retaining clip 501 in the open configuration, it may be removed from the extendible member 50.

The retaining clip 501 could take a number of different forms. For example, the retaining clip 501 may be in the form of a U-shaped member defining a slit between two prongs. The extendible member 50 may be received by the slit, when may be narrower than a diameter of the extendible member 50 to clamp the extendible member 50 between the two prongs of the retaining clip 501.

In some embodiments of the retaining clip 501, the slit narrows with depth between the prongs so that the clip 501 may be pushed onto the extendible member 50 to clamp the member 50 between the prongs.

In some embodiments, therefore, the elongate members 501a, 501b are not coupled by a hinge and may form the aforementioned prongs.

In some embodiments, the engagement mechanism may be such that the retaining clip 501 has to be cut to allow removal from the extendible member 50. For example, the engagement mechanism may include the welding, e.g. plastic welding, of the two elongate members 501a,501b to each other. This may, for example, prevent reuse of the retaining clip 501.

In some embodiments, there is provided a valve system 100 in combination with an extendible member 50 and a retaining clip 501. In some embodiments, this combination may include the insertion tool 200 and may, therefore, be referred to as a valve kit (the kit including the means by which the valve system 100 may be inserted or otherwise implanted). In some embodiments, the valve kit may include an insertion tool 300 of a different form (e.g. in a form described herein below).

The valve system 100 may include one or more tethers 101 for use in extracting the valve system 100 from a patient. The or each tether 101 may include a thread or wire which extends downwardly (relative to the normal direction of fluid flow) through the urethra and which may extend out of the patient.

For extraction, the or each tether 101 may be grasped (e.g. using an appropriate clamping tool) and used to pull the valve system 100 downwardly (relative to the normal direction of fluid flow) through the urethra. This may collapse the retention mechanism to allow this to pass through the urethra. The or each tether 101 may be sufficiently strong to permit this to occur. The or each tether 101 may be of sufficient length to be externally or nearly externally accessible through the urethra.

In some embodiments, the or each tether 101 is secured to the valve 1. The or each tether 101 may be secured to or around at least part of the ferromagnetic ring 5 and/or the valve seat member 41, for example. This may help to prevent, for example, the or each tether 101 cutting through the material of the valve main body 11 during extraction of the valve system 100. In some embodiments, the or each tether 101 may include at least a part which is helically wound and embedded within the valve main body 11 (such that part of the or each tether 101 may extend around the valve main body 11 a plurality of times) - again, this may help to prevent the tether 101 from cutting through the material of the valve main body 11 during extraction of the valve system 100.

The valve system of the prior art commonly locates the valve in the bulbous urethra 26. The valve system 100 of embodiments may be similarly formed such that the valve 1 is located in the bulbous urethra 26. However, in some embodiments, the valve 1 may be located in the pendulous urethra 27 and the valve system 100 may be correspondingly configured (e.g. with respect to the length of the catheter 16).

Location of the vale in the pendulous urethra 27 may have one or more advantages, such as making actuation of the valve with the magnetic member 15 easier and/or reducing pressure pain caused by the patient sitting down when the valve 1 is located higher in the urethra.

Facilitation of the location of the valve 1 in the pendulous urethra 27 may be assisted due to the lower bulk and/or increased comfort sought by some embodiments.

In some embodiments, with the valve 1 located in the pendulous urethra 27, there may be additional external seal members 113 located along the catheter 16. These additional external seal members 113 may be located at one or more of the bladder neck 22, the prosthetic urethra 23, the external sphincter 24, and the bulbous urethra 26, for example. In some embodiments, the or each additional external seal member 113 may be generally of the same form as described above in relation to the external seal members 113 and may be similarly constructed (e.g. out of the same materials as described above and/or of annular form). In some embodiments, the or each external seal member 113 located along a length of the catheter 16 and/or the valve 1 may have one or more different sealing properties.

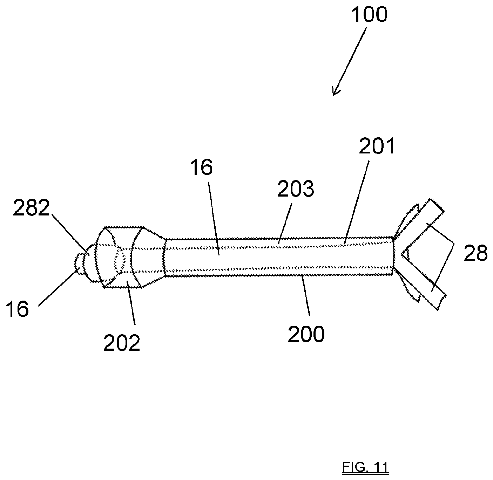

Some embodiments may be configured for implantation into female urethra and an example of a valve system 100 for female patients can be found in figure 11. Such a valve system 100 for female patients may include a retention mechanism for locating in the bladder 29, a catheter 16 extending through the urethra and a valve 1. Due to the shorter length of the female urethra, the catheter 16 may be shorter than would be the case for a valve system 100 for a male patient.

In some embodiments, the valve system 100 for a female urethra may have a valve 1 which is not located at a distal end of the catheter 16 (as is the case in the above described embodiments) but which is located along a length of the catheter 16. In practice, this may be achieved by having a first catheter which extends from the retention mechanism to the valve 1 (and, in particular, the inlet port 112 of the valve 1) and a second catheter which extends from the valve 1 (and, in particular, the outlet port 25 of the valve 1) to a distal end of the valve system 100.

In some embodiments, the valve system 100 may include a second retention mechanism which may be referred to as an external retention mechanism 282. Whilst the (first) retention mechanism at the proximal end of the valve system 100 may be configured to reduce the risk of the valve system 100 migrating out of position down the urethra (relative to the normal flow of fluid therethrough), the external retention mechanism 282 may be configured to reduce the risk of the valve system 100 migrating into the bladder 29.

Therefore, in some embodiments of the valve system 100 (which may be embodiments intended for female patients), the valve system 100 may have a respective retention mechanism at each opposing end of the valve system 100.

In some embodiments, the first retention mechanism (for fitting inside the bladder 29, which may referred as an internal retention mechanism) may be of a similar form to that described in the above embodiments and may, therefore, include a plurality of deployable members 28 which are configured to be moved between an undeployed (or retracted) configuration to a deployed (or extended) configuration. The deployable members 28 may in the form of arms or loops or rings 28, for example. In the depicted example of figure 11 the deployable members 28 are in the form of arms. The first retention mechanism may be in the form of a malecot.

The external retention mechanism 282 may have a different form to the first retention mechanism. In some embodiments, the external retention mechanism 282 may include an adjustable retention ring which may be configured to be located at the urethral exit between the labial folds. The retention ring may, therefore, be adjustable to fit between the labial folds and but sufficiently large to reduce the likelihood of the retention ring entering the urethra. The adjustable retention ring may be in the form of a drawstring around a neck of the catheter 16 at the distal end thereof.

In some embodiments, the distal end of the catheter 16 may extend through the adjustable retention ring and may be of a concentric form.

In some embodiments, the catheter 16 and/or the external retention mechanism 282 may be configured to be cut to adjust the length of the valve system 100.

The external retention mechanism 282 may include a foam pad (which may be formed of foam material as described herein and which may carry antimicrobial agents). In some embodiments the foam pad may be configured to be cut to a desired size and/or shape (and may include one or more guide lines (e.g. printed thereon for this purpose)).

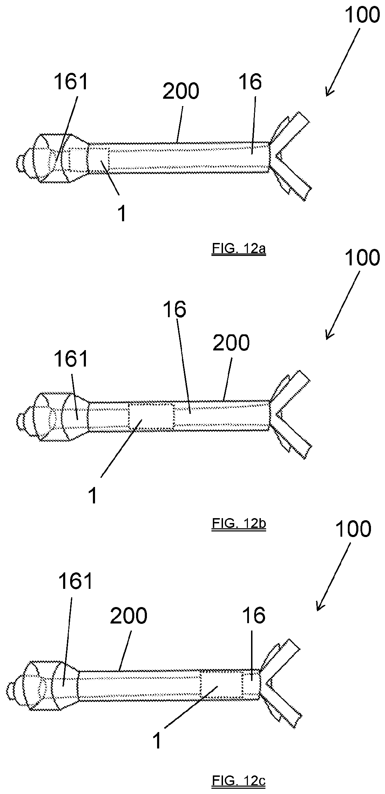

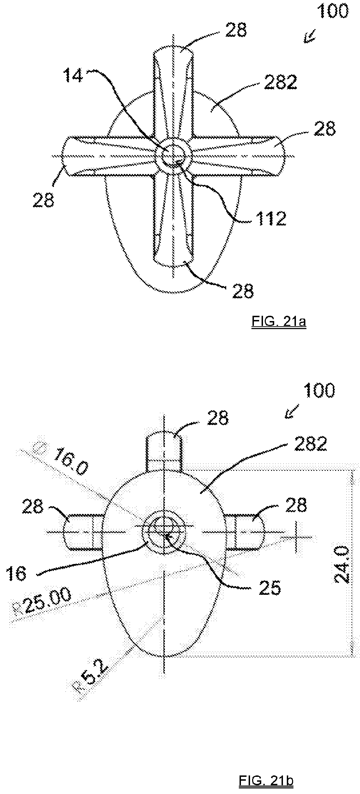

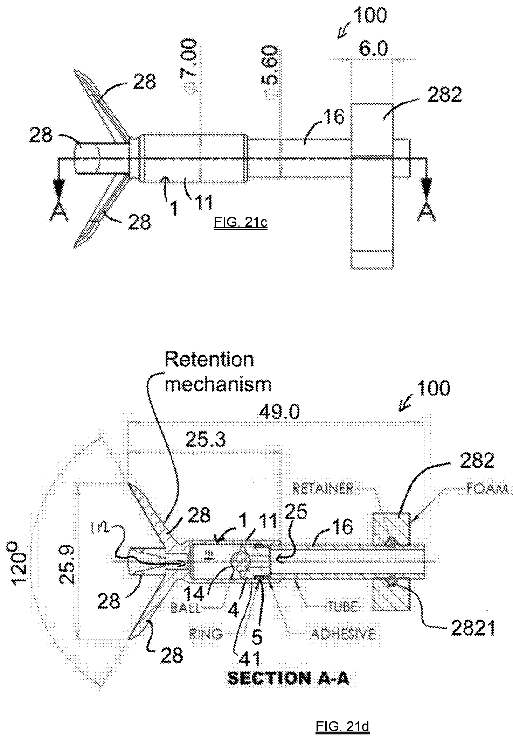

Another embodiment is shown in figures 21a-21d. This embodiment is a variation of the embodiments described with reference to figures 11 and 12a-12c, for example.

In this embodiment, various dimensions are provided in the figures by way of example only. The dimensions are indicated in mm.

In the some embodiments, including the depicted embodiment of figures 21a-21d, there may be a plurality of retention members 28 (e.g. two, three, four (as depicted), five, or more).

The second retention mechanism 282 may include a foam member (or pad), for example. The foam member may be held in place through an annular retainer 2821 located around at least part of the catheter 16. The retainer 2821 may be adhered to the catheter 16, for example. An outer surface of the annular retainer 2821 may be keyed to fit a corresponding recess in the second retention mechanism 282. The second retention mechanism 282 may be adhered, for example, to the annular retainer 2821. The second retention mechanism 282 may be offset with respect to the catheter 16. The second retention mechanism 282 may be generally oval in shape, for example.

The valve seat 4 in the embodiments described with reference to figures 21a-21d may be a valve seat 4 as described elsewhere herein and may be part of a valve seat member 41.

The ferromagnetic ring 5 in the embodiments described with reference to figures 21a-21d may be a ferromagnetic ring 5 as described elsewhere herein. For example, the ferromagnetic ring 5 may be in the form of an annular collar, for example. The ferromagnetic ring 5 may extend around at least part of the valve seat member 41, for example. The ferromagnetic ring 5 may be surrounded by at least part of the valve main body 11 and may fit within the valve main body 11.

The valve main body 11 may be at least partially tubular in form such that the valve seat 4 fits within the valve main body 11. The catheter 16 may also be at least partially received by the valve main body 11 and may be adhered thereto such that the outlet port 16 is in fluid communication with the catheter 16.

The ferromagnetic ring 5 may be adhered to the valve seat 4, and/or may be held in place by the catheter 16 (which may be adhered to the valve main body 11).

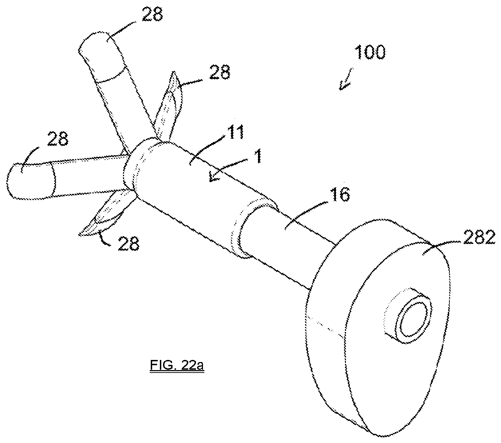

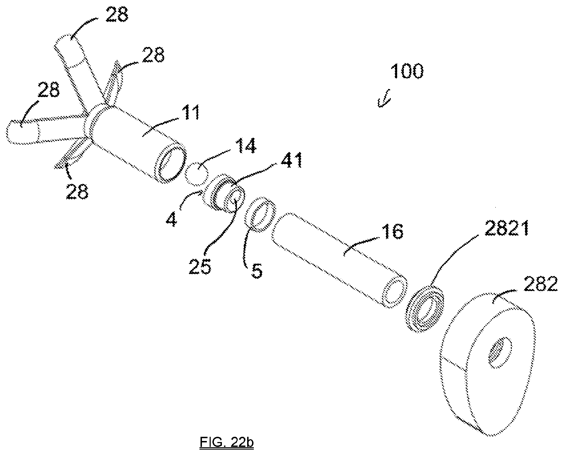

Figures 22a and 22b show different views of the embodiments described with reference to figures 21a-21d.

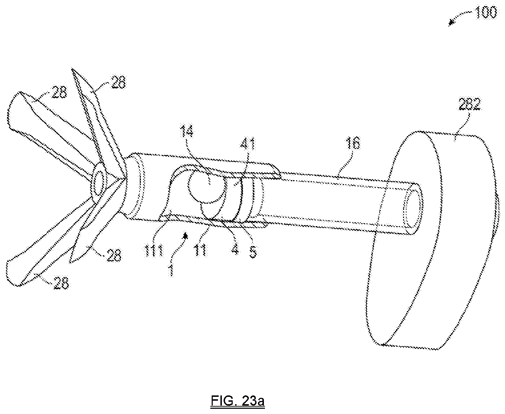

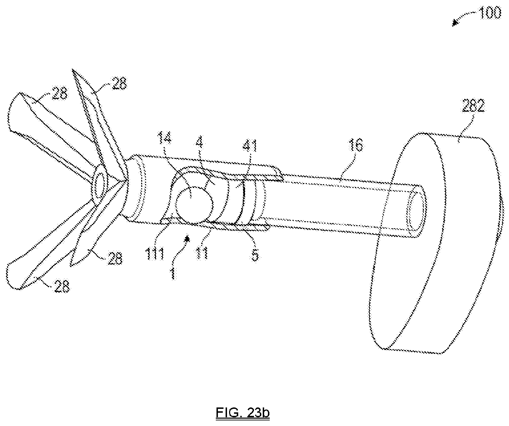

Figure 23a shows the embodiment of figures 21a-21b with the valve 1 in the open configuration. Figure 23b shows the same embodiment with the valve 1 in the closed configuration.

In some embodiments, there may be one or more external seal members 113 at or towards the distal end of the valve system 100 of these embodiments. The or each external seal member 113 may include a seal member located at the external retention mechanism 282. The or each external seal member 113 may be generally of the same form as those described above and may be configured to reduce the risk of leakage between the valve system 100 and the urethra wall. The or each external seal member 113 may include a generally annular external seal member 113 which is located between the external retention mechanism 282 and a position adjacent the urethra. This may be provide some sealing effect but also may reduce irritation at this site. One or more other external seal members 113 may be located along the length of the catheter 16 (which may be the first or second catheter, in some embodiments). These other external seal members 113 may each be configured to assist in reducing the likelihood of leaks between the valve system 100 and the urethra wall.

Reference has been made to the location of the valve 1 along the length of the catheter 16 and the provision of first and second catheters either side of the valve 1. Figure 11 does not, however, depict the valve 1. Some example valve 1 locations are shown, therefore, in figures 12a-12c.

In figure 12a, the valve 1 is located adjacent the distal end of the valve system 100 and close to the external retention mechanism 282 ("close" may mean closer to the external retention mechanism 282 than to the first retention mechanism, for example).

In figure 12b, the valve 1 is located approximately midway between the external retention mechanism 282 and the first retention mechanism.

In figure 12c, the valve 1 is located adjacent the proximal end of the valve system 100 and close to the first retention mechanism ("close" may mean closer to the first retention mechanism than to the external retention mechanism 282, for example).

Figures 11 and 12a-12c all depict the valve system 100 with an insertion tool 200 which may be used in relation to some embodiments. This insertion tool 200 may be configured for use with shorter valve systems 100 such as those for female patients. The insertion tool 200 may differ, therefore, from the insert tool 200 described above in relation to some other embodiments.

The insertion tool 200 may, in some embodiments, include a relatively rigid sleeve member 201. This sleeve member 201 may be more rigid than the valve system 100 so as to enable the pushing of the insertion tool 201 into the urethra and therethrough. The sleeve member 201 may be tubular in form defining a lumen and this lumen may be configured to receive the valve system 100 (or a part thereof). The insertion tool 200 may have a neck section 202 which has lumen width or diameter which is generally larger than a lumen width or diameter of a main section 203 of the insertion tool 200. An internal and/or external diameter of the insertion tool 200 may taper towards one or both ends thereof.

The insertion tool 200 may be inserted into the urethra without the valve system 100 received therein. The valve system 100 may then be passed through the insertion tool 200 until the first retention mechanism deploys within the bladder 29. The insertion tool 200 may then be removed and any necessary adjustment made to the external retention mechanism 282. Alternatively, the insertion tool 200 may be provided with the valve system 100 provided at least partially therein. The combination of the insertion tool 200 and valve system 100 may then be inserted into the urethra and the same process followed. This latter option may enable a more flexible insertion tool 200 to be used, which may reduce the risk of damage to the urethra wall, for example. Embodiments may include the insertion tool 200 in combination with the valve system 100, for example.

Some embodiments have been described as including an external seal member 113. One or more such external seal members 113 may be provided not only in relation to the valve 1 but also (or alternatively) in relation to the catheter 16. This may include, for example a coating over a substantial part of the external surface of the catheter 16 and/or the valve 1. In some embodiments, there is a plurality of external seal members 113 which form circumferential ribs around parts of the valve system 100. In some embodiments, a coating material forming an external seal member 113 may include one or more circumferential ribs of material along a length of the valve system 100.

In some embodiments, the or each external seal member 113 may be configured to expand when exposed to a liquid (such as to water), so as to improve the seal between the valve system 100 (or part thereof) and the urethra wall. The or each external seal member 113 may be formed of a material as described above. In some embodiments, the or each seal member may be formed from a hydrogel material or other polymer gel material. This material may be a silicone material. In some embodiment, the or each external seal member 113 may be formed from CRM (cotton rayon mixes), e.g. as used in TAMPAX (RTM) tampons, or compressed Gelfoam(RTM).

The material used for the or each external seal member 113 may include antimicrobial and/or antifungal agents which may be impregnated into the material, for example.

The material used for the or each external seal member 113 may include an anaesthetic agent which may be impregnated into the material, for example. Such an anaesthetic may aid in reducing discomfort and especially discomfort immediately after implantation.

Also as discussed herein, one of the external seal members 113 may differ in form and/or material from another of the external seal members 113.

In some embodiments, the or each external seal member 113 may include a material which changes colour on exposure to one or more microorganism types and/or their secretions. This may enable assessment of the impact of such microorganisms on extraction of the valve system 100, for example. In some embodiments, this may then determine what antimicrobial and/or antifungal agents to use in relation to a new valve system 100 for the same patient, for example.

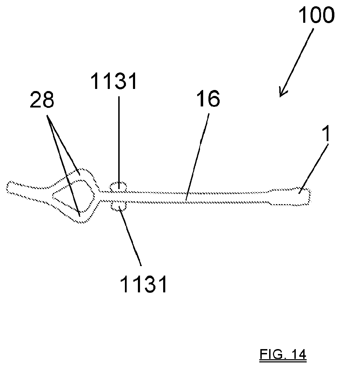

In some embodiments, the or each external seal member 113 may include an inflatable member 1131 (see figure 14). The inflatable member 1131 may be inflatable with a saline solution or silicone or a polymer gel or another fluid. The inflation fluid for the or each inflatable external seal member 1131 may be provided via a tube which may extend along an outer surface of at least part of the valve system 100 or which may extend through at least part of the valve system 100 (e.g. through the valve 100 and/or the catheter 16. The or each inflatable external seal member 1131 may include a valve system to retain the inflation fluid and allow removal of the tube through which the inflation fluid was provided. In some embodiments, the or each inflatable external seal member 1131 is a sealed member which may be compressed for insertion into the urethra - e.g. by a restriction band - the compression may be removed - e.g. by removal of the restriction band - following insertion to the desired location. Removal of the compression may allow expansion of the inflatable external seal member 1131.

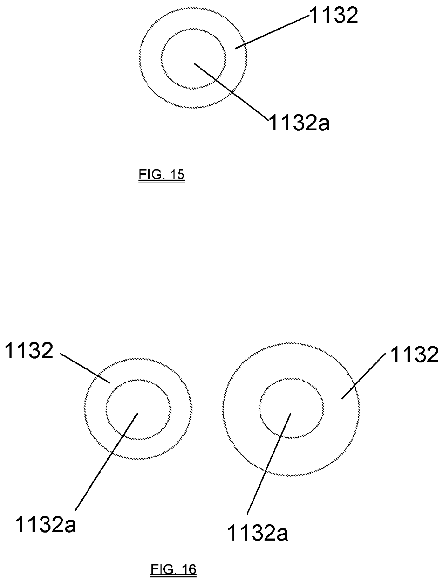

In some embodiments, one or more external seal members 113 may be provided which may be secured to the valve system 100 with the valve system 100 implanted. In some embodiments, the or each such external seal member 113 may include an annular member 1132 (see figures 15 and 16, for example) which may be slid along a length of at least part of the valve system 100. There may be a plurality of such external annular seal members 1132 which may have different external diameters such that the external seal member 113 which provides the best seal for a particular patient may be used - see figure 16 which shows two such members 1132. In some embodiments, an internal diameter (or other dimension - see the dimension of the hole 1132a defined by each member 1132) of each such external seal member 1132 may be configured to provide a close fit with part of the valve system 100. In some embodiments, the or each such external seal member 1132 may be configured to expand when exposed to a liquid (see the above description) and this may allow the external seal member 1132 to be more easily slid into place before expansion seals the member 1132 to the valve system 100. In some embodiments, internal diameters (or other dimensions) of a set of external seal members 1132 may be such that a first external seal member 1132 is configured to seal against part of the valve system 100 and a second external seal member 1132 is configured to seal against the first external seal member 1132, and so on. As such, an internal diameter (or other dimension) in a set of external seal members 1132 may be progressively larger and the external diameter may likewise be progressively larger (in some embodiments, an internal diameter of the second external seal member 1132 matches (e.g. is substantially equal to) an external diameter of the first external seal member 1132, and this pattern may be repeated throughout the set). Accordingly, a set of external seal members 1132 may be used to increase the external dimension of the seal progressively, with the addition of each external seal member 113 in the set until the desired seal has been achieved. A sleeve like tool may be provided to push each such external seal member 1132 to the desired location.

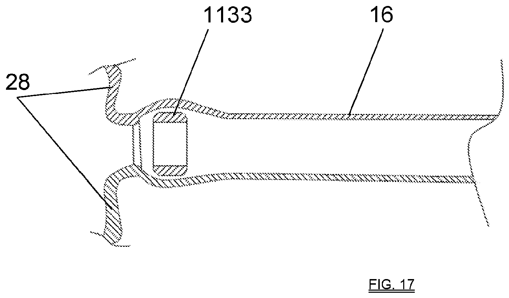

In some embodiments, one or more external seal members 1132 may be provided which may be secured to the valve system 100 with the valve system 100 implanted as described above but which may be secured from the proximal end of the valve system 100 and pulled or pushed into position using a tool (which may a hooked tool) from the distal end thereof. In some embodiments, instead of one or more such external seal members 113, a similar effect may be achieved by inserting (after implantation) one or more annular members 1133 (see figure 17, for example) within the catheter 16 at the end thereof adjacent the retention mechanism (i.e. at the proximal end). Such annular members 1133 may allow the flow of fluid therethrough but may press a wall of the catheter 16 outwardly to provide a rib-like bulge in an external surface thereof. Members other than annular members 1133 may be used for this purpose - e.g. a cage member may be used (e.g. a mesh sphere).

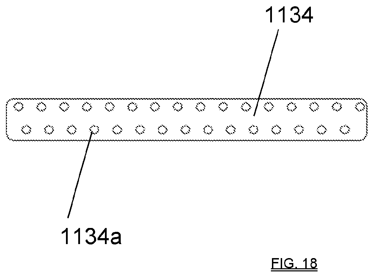

In some embodiments, the or each external seal member 113 may include a sleeve member 1134. The sleeve member 1134 may be configured to be located around at least part of the valve system 100 and may be secured thereto (e.g. with an interference fit and/or an adhesive). The sleeve member 1134 may extend along at least part of a length of the valve system 100. In some embodiments, the sleeve member 1134 may extend between two other forms of external seal member 113 (such as those described herein). A plurality of sleeve members 1134 may be provided. The or each sleeve member 1134 may include one or more areas 1134a on its outer surface which are of a different material and/or texture then one or more other areas. In some embodiments, the or each sleeve member 1134 may include one or more holes therethrough, such that that the or each sleeve member 1134 is a perforated sleeve member 1134. The or each sleeve member 1134 may be configured to reduce the risk of adhesion between the urethra wall and the valve system 100. The or each hole or area of different material/texture 1134a may be provided in a pattern - which may include rib-like circumferential bands and/or rib-like axial lengths and/or patches or spots (as shown in figure 18, for example).

One or more parts of the valve system 100 may have a larger external dimension (e.g. diameter) then one or more other parts. One or more parts of larger external dimension may be configured to correspond with one or more parts of the patient in which the valve system 100 is to be implanted. For example, the external dimension of the valve system 100 past the external sphincter 24 may be larger than above the external sphincter 24 (with respect to the normal direction of fluid flow). The larger external diameter may be provided be the catheter 16 and/or valve 1 and/or one or more external seal members 113 as described herein.

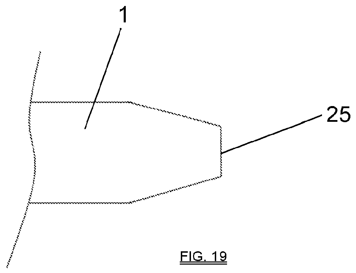

In some embodiments, the outlet port 25 of the valve 1 may be at least partially defined by a tapered end portion (see figure 19, for example) - which may be an end portion of the valve main body 11 or a plug defining the outlet port 25. This may help to reduce the risk of blockage of the outlet port 25 and/or ingrowth into the valve 1.

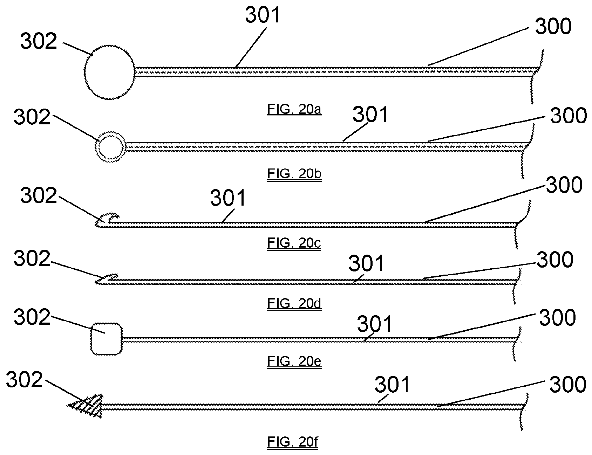

In some embodiments, there is provided a different form of insertion tool 300 to those described above - either in relation to the prior art or in relation to the embodiments described with reference to figures 11 and 12. Examples of this insertion tool 300 are shown in figures 20a-20l.

This insertion tool 300 may include a main body 301 which is at least as rigid as the valve system 100. The main body 301 may be sufficiently rigid to allow the main body 301 to push the valve system 100 to the desired implantation location. The main body 301 may be generally elongate (such that it may fit through a human urethra). In some embodiments, the rigidity of the main body 301 may vary along its length to provide one or more parts which are more rigid than others (this may be achieved by changes in the cross-sectional size and/or shape along portions of the main body 301). At a proximal end of the main body 301 there is a valve system attachment member 302. The valve system attachment member 302 is configured to engage at least part of the valve system 100 and this part may be the valve 1. In some embodiments, the valve system attachment member 302 is configured to engage the valve 1 at the outlet port 25 and may extend (when engaged) at least partly into the outlet port 25 or around a part of the external surface of the valve 1.

The engagement of the valve system attachment member 302 is such that the valve system 100 may be pushed by the insertion tool 300 during implantation and such that some pulling of the valve system 100 in the opposing direction is also possible.

The valve system attachment member 302 could take a number of different forms.

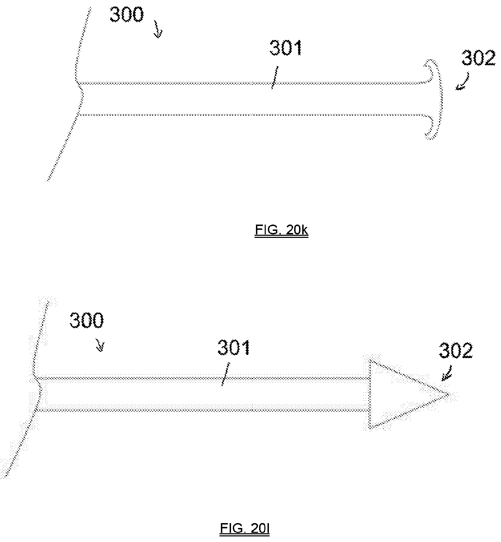

In some embodiments (see figure 20d or 20c, for example), the valve system attachment member 302 may include a hook (which may be a barbed hook) which is configured to engage with the material of the valve main body 11 and/or a plug located towards the outlet port 25. Figure 20l shows a variation of the hook embodiment but may be conical in form. The valve system attachment member 302 may pass through the outlet port 25 into the internal valve volume 111 to engage an inner part thereof - such as the valve seat or a part thereof.

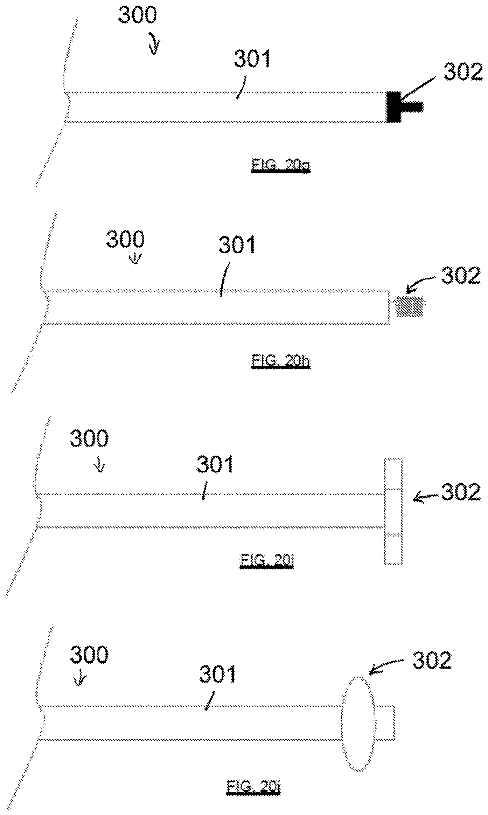

In some embodiments (see figure 20f, for example), the valve system attachment member 302 may include a helical thread (and the attachment member 302 may be tapered) which is configured to engage with the material of the valve main body 11 and/or a plug located towards the outlet port 25. Figure 20h is a variation of the embodiment of figure 20f, for example. In this embodiments, the valve system attachment member 302 may be in the firm of a helically wound wire. The helically wound wire may be configured to engage with the material of the valve main body 11 and/or may pass through the outlet port 25 by rotation thereof.

In some embodiments (see figure 20d, for example), the valve system attachment member 302 may include a hinged hook (which may be a barbed hinged hook) which is configured to engage with the material of the valve main body 11 and/or a plug located towards the outlet port 25 but which is also configured to rotate on disengagement to lie along a longitudinal axis of the main body of the insertion tool (to aid extraction of the tool without damage to the urethra wall).

In some embodiments (see figure 20e, for example), the valve system attachment member 302 may include a magnetic engagement member which is configured to engage, through magnetic attraction, the valve element 14 and/or the ferromagnetic ring 5 (to do so the magnetic engagement member may extend through the outlet port 25 into the valve 1, for example). Figure 20g shows a variation of the embodiment of figure 20e, for example. In this embodiment, the magnetic engagement member may be shaped to pass through the outlet port 25, at least partially. Accordingly, the magnetic engagement member may include a cylindrical portion shaped and sized to be at least partially received by the outlet port 25.

In some embodiments (see figure 20b, for example), the valve system attachment member 302 may include a deployable member (which may include one or more arms, loops or rings) which is configured to be deployed within the internal valve volume 111 such that the deployable member cannot be removed through the outlet - the valve system attachment member 302 extending through the outlet 25 into the valve 1. The deployable member may be actuatable into an undeployed state selectively - to achieve this the deployable member may be coupled to a wire which extends through the main body 301 of the insertion tool to the distal end thereof, wherein pulling on the wire causes the deployable member to retract at least partially into the valve system attachment member 302 and/or the main body 301 of the insertion tool 300 (both of which may be at least partially tubular in form). The deployable member 302 may comprise one or more resilient members, for example.

Figure 20i depicts another example embodiment in which the valve system attachment member 302 includes a deployable member or members. In particular, the valve system attachment member 302 may include one or more deployable wings (two are depicted). The or each wing may be located so as, in the deployed state (as depicted) to extend radially outwardly from a longitudinal axis of the insertion tool 300. In a retracted state, the or each deployable member (e.g. wing) may be retractable (e.g. to a position to rest along a length of the main body 301. The or each deployable member may be placed in the retracted state for insertion through the urethra, for example. The or each deployable member may be placed in the deployed state with the or each deployable member within the internal valve volume 111 (i.e. past the valve seat). The or each deployable member may be a hinged member and actuation may be controlled via one or more cables (which may extend through the main body 301). The or each deployable member may have a hook-like form - such as depicted in figure 20k, for example.

In some embodiments, the or each deployable member may be configured to adopt the deployed state on movement of the outwardly through the outlet port 25, for example.

Figure 20j is a variation of the embodiment of figure 20b, in this embodiment, the deployable member may comprise an inflatable member such as a balloon which is inflatable using an inflation fluid (such as saline solution) which may be delivered to (and withdrawn from) the inflatable member through the main body 301. Inflation may occur with the inflatable member within the internal valve volume 111.

In a similar manner, in some embodiments (see figure 20a, for example), the valve system attachment member 302 may include a retractable loop which is configured to engage an external surface of the valve 1, wherein the loop can be expanded or retracted using a portion of the material of the loop which extends to the distal end of the insertion tool 300 (e.g. through a part of the insertion tool 300).

As will be appreciated, the valve system attachment member 302 may be configured into a deployed state with a first diameter and a retracted state with a second diameter - the first diameter being greater than the second diameter. The deployed state may be used to engage the valve 1 and the retracted state may be used for insertion of the tool 300 into the urethra, for example.

As will be appreciated, a distal end of the tool 300 may be manually manipulated to insert or extract the valve system 100.

The insertion tool 300 described above may be used as an extraction tool 300 and may be a dual purpose insertion and extraction tool 300.

In some embodiments, the elastomer described herein may be silicone. Silicone may be used to form the valve main body 11, and/or the valve seat 4 and/or the valve seat member 41.

Other elastomeric polymers may be used in some embodiments.

The valve 1 and/or other parts of the valve system 100 may be coated in antimicrobial and/or antifungal and/or lubricious materials. This may be achieved by virtue of the external seal member 113 or otherwise (e.g. in addition to an external seal member 113) - such as described herein. As will be appreciated, the catheter 16 may be coated in antimicrobial and/or antifungal and/or lubricious materials. For example, an external surface - which may be substantially the entire external surface - of the catheter 16 may be so coated. In some embodiments, the internal surface(s) of the catheter 16 are also (or alternatively) so coated. Accordingly, in some embodiments, substantially the entire catheter 16 may be coated in this manner.

In some embodiments, the valve main body 11 may, in combination with the inlet 112a and/or outlet 25a tubular plug form a valve main body arrangement.

When used in this specification and claims, the terms "comprises" and "comprising" and variations thereof mean that the specified features, steps or integers are included. The terms are not to be interpreted to exclude the presence of other features, steps or components.

## Claims

1. An intraurethral magnetic valve (1) having an inlet port (112) and an outlet port (25), the valve including:
a valve main body (11);
a ferromagnetic ring (5) located at least partially within the valve main body (11);
a valve seat (4); and
a magnetic valve element (14) located within an internal volume (111) defined by the valve main body (11) and configured for movement with respect to the ferromagnetic ring (5), that movement being within the valve main body (11) between a closed configuration in which fluid is inhibited from flowing from the inlet port (112) through the outlet port (25) and an open configuration in which fluid is permitted to flow from the inlet port (112) through the outlet port (25), movement of the magnetic valve element (14) being on exposure to a magnetic field from an external magnet member, wherein the valve seat (4) is formed from an elastomer material,
**characterized in that** the valve seat (4) is an offset valve seat such that the outlet port (25) is offset from a central longitudinal axis of the internal volume (111) defined by the valve main body (11).

2. A valve (1) according to claim 1, wherein an elastomer material of the valve main body (11) forms the valve seat (4) with which the magnetic valve element (14) is configured to engage when in the closed configuration.

3. A valve (1) according to claim 1, further comprising a valve seat member (41) defining the valve seat (4) and fitted to the valve main body (11), optionally the valve seat member (41) is formed of only elastomer material or materials.

4. A valve (1) according to claim 3, wherein the valve seat member (41) is at least partially received by the valve main body (11).

5. A valve (1) according to claim 2 or 3, wherein the ferromagnetic ring (5) at least partially surrounds a portion of the valve seat member (41).

6. A valve (1) according to any preceding claim, wherein the ferromagnetic ring (5) is embedded in the elastomer material which forms the valve seat (4).

7. A valve (1) according to any preceding claim, further including an external seal member (113) configured to surround at least a part of the valve main body (11) to aid in providing a seal between the valve (1) and a urethral wall, optionally wherein the external seal member (113) includes a foamed material, and optionally wherein the external seal member (113) includes an antimicrobial and/or antifungal agent.

8. A valve (1) according to any preceding claim, wherein only the magnetic valve element (14) and ferromagnetic ring (5) are not elastically deformable.

9. A valve (1) according to any preceding claim, wherein the valve main body (11) is tapered towards the outlet port (25).

10. A valve system (100) including a valve (1) according to any preceding claim, a catheter (16) coupled to the inlet port (112) of the valve (1), and a retention mechanism (28) coupled to the catheter (16), wherein the retention mechanism (28) is configured for deployment in a bladder.

11. A valve system (100) according to claim 10, further including:
an extendible element (50) extending through the valve (1) and catheter (16), and secured to at least part of the retention mechanism (28); and
a retaining clip (501) configured to engage the extendible element and inhibit the movement thereof through the catheter (16).

12. A valve system (100) according to claim 10 or **11,** further including an external retention mechanism (282) configured to inhibit migration of the valve system (100) into the bladder.

13. A valve system (100) according to any of claims 10 to 12, further including a second catheter (16) which couples the external retention mechanism (282) to the valve (1).

## Patentansprüche

1. Intraurethrales Magnetventil (1) mit einer Einlassöffnung (112) und einer Auslassöffnung (25), wobei das Ventil Folgendes enthält:
einen Ventilhauptkörper (11);
einen ferromagnetischen Ring (5), der sich mindestens teilweise innerhalb des Ventilhauptkörpers (11) befindet;
einen Ventilsitz (4); und
ein magnetisches Ventilelement (14), das sich innerhalb eines Innenvolumens (111) befindet, das durch den Ventilhauptkörper (11) definiert ist, und für eine Bewegung in Bezug auf den ferromagnetischen Ring (5) konfiguriert ist, wobei diese Bewegung innerhalb des Ventilhauptkörpers (11) zwischen einer geschlossenen Konfiguration, in der verhindert wird, dass Fluid von der Einlassöffnung (112) durch die Auslassöffnung (25) strömt, und einer offenen Konfiguration, in der ermöglicht wird, dass Fluid von der Einlassöffnung (112) durch die Auslassöffnung (25) strömt, erfolgt, wobei Bewegung des magnetischen Ventilelements (14) bei Exposition gegenüber einem Magnetfeld von einem externen Magnetelement erfolgt, wobei der Ventilsitz (4) aus einem Elastomermaterial ausgebildet ist,
**dadurch gekennzeichnet, dass** der Ventilsitz (4) ein versetzter Ventilsitz ist, sodass die Auslassöffnung (25) von einer zentralen Längsachse des Innenvolumens (111), das durch den Ventilhauptkörper (11) definiert ist, versetzt ist.

2. Ventil (1) nach Anspruch 1, wobei ein Elastomermaterial des Ventilhauptkörpers (11) den Ventilsitz (4) ausbildet, mit dem in Eingriff zu gelangen das magnetische Ventilelement (14) konfiguriert ist, wenn es sich in der geschlossenen Konfiguration befindet.

3. Ventil (1) nach Anspruch 1, ferner umfassend ein Ventilsitzelement (41), das den Ventilsitz (4) definiert und an dem Ventilhauptkörper (11) angebracht ist, wobei das Ventilsitzelement (41) optional nur aus Elastomermaterial oder -materialien ausgebildet ist.

4. Ventil (1) nach Anspruch 3, wobei das Ventilsitzelement (41) mindestens teilweise durch den Ventilhauptkörper (11) aufgenommen ist.

5. Ventil (1) nach Anspruch 2 oder 3, wobei der ferromagnetische Ring (5) mindestens teilweise einen Abschnitt des Ventilsitzelements (41) umgibt.

6. Ventil (1) nach einem vorhergehenden Anspruch, wobei der ferromagnetische Ring (5) in das Elastomermaterial eingebettet ist, das den Ventilsitz (4) ausbildet.

7. Ventil (1) nach einem vorhergehenden Anspruch, ferner enthaltend ein externes Dichtungselement (113), das dazu konfiguriert ist, mindestens einen Teil des Ventilhauptkörpers (11) zu umgeben, um ein Bereitstellen einer Dichtung zwischen dem Ventil (1) und einer Harnröhrenwand zu unterstützen, wobei das externe Dichtungselement (113) optional ein geschäumtes Material enthält und wobei das externe Dichtungselement (113) optional ein antimikrobielles und/oder antimykotisches Mittel enthält.

8. Ventil (1) nach einem vorhergehenden Anspruch, wobei nur das magnetische Ventilelement (14) und der ferromagnetische Ring (5) nicht elastisch verformbar sind.

9. Ventil (1) nach einem vorhergehenden Anspruch, wobei sich der Ventilhauptkörper (11) zu der Auslassöffnung (25) hin verjüngt.

10. Ventilsystem (100), enthaltend ein Ventil (1) nach einem vorhergehenden Anspruch, einen Katheter (16), der mit der Einlassöffnung (112) des Ventils (1) gekoppelt ist, und einen Rückhaltemechanismus (28), der mit dem Katheter (16) gekoppelt ist, wobei der Rückhaltemechanismus (28) zum Einsatz in einer Blase konfiguriert ist.

11. Ventilsystem (100) nach Anspruch 10, ferner enthaltend:
ein ausfahrbares Element (50), das sich durch das Ventil (1) und den Katheter (16) erstreckt und an mindestens einem Teil des Rückhaltemechanismus (28) befestigt ist; und
einen Rückhalteclip (501), der dazu konfiguriert ist, mit dem ausfahrbaren Element in Eingriff zu treten und dessen Bewegung durch den Katheter (16) zu verhindern.

12. Ventilsystem (100) nach Anspruch 10 oder 11, ferner enthaltend einen externen Rückhaltemechanismus (282), der dazu konfiguriert ist, ein Wandern des Ventilsystems (100) in die Blase zu verhindern.

13. Ventilsystem (100) nach einem der Ansprüche 10 bis 12, ferner enthaltend einen zweiten Katheter (16), der den externen Rückhaltemechanismus (282) mit dem Ventil (1) koppelt.

## Revendications

1. Valve magnétique intra-urétrale (1) présentant un orifice d'entrée (112) et un orifice de sortie (25), la valve comprenant :
un corps principal de valve (11) ;
une bague ferromagnétique (5) située au moins partiellement au sein du corps principal de valve (11) ;
un siège de valve (4) ; et
un élément de valve magnétique (14) situé au sein d'un volume interne (111) défini par le corps principal de valve (11) et conçu pour se déplacer par rapport à la bague ferromagnétique (5), ce mouvement étant au sein du corps principal de valve (11) entre une configuration fermée où du fluide ne peut pas s'écouler en provenance de l'orifice d'entrée (112) à travers l'orifice de sortie (25) et une configuration ouverte où du fluide peut s'écouler à partir de l'orifice d'entrée (112) à travers l'orifice de sortie (25), le mouvement de l'élément de valve magnétique (14) étant exposé à un champ magnétique en provenance d'un élément magnétique externe, dans laquelle le siège de valve (4) est formé à partir d'un matériau élastomère,
**caractérisé en ce que** le siège de valve (4) est un siège de valve décalé de sorte que l'orifice de sortie (25) soit décalé par rapport à un axe longitudinal central du volume interne (111) défini par le corps principal de valve (11).

2. Valve (1) selon la revendication 1, dans laquelle un matériau élastomère du corps principal de valve (11) forme le siège de valve (4) avec lequel l'élément de valve magnétique (14) est conçu pour venir en prise lorsqu'il est dans la configuration fermée.

3. Valve (1) selon la revendication 1, comprenant en outre un élément de siège de valve (41) définissant le siège de valve (4) et monté sur le corps principal de valve (11), éventuellement l'élément de siège de valve (41) est formé uniquement d'un matériau ou de matériaux élastomères.

4. Valve (1) selon la revendication 3, dans laquelle l'élément de siège de valve (41) est au moins partiellement reçu par le corps principal de valve (11).

5. Valve (1) selon la revendication 2 ou 3, dans laquelle la bague ferromagnétique (5) entoure au moins partiellement une partie de l'élément de siège de valve (41).

6. Valve (1) selon une quelconque revendication précédente, dans laquelle la bague ferromagnétique (5) est encastrée dans le matériau élastomère qui forme le siège de valve (4).

7. Valve (1) selon une quelconque revendication précédente, comprenant en outre un élément d'étanchéité externe (113) conçu pour entourer au moins une pièce du corps principal de valve (11) pour contribuer à fournir une étanchéité entre la valve (1) et une paroi urétrale, éventuellement dans laquelle l'élément d'étanchéité externe (113) comprend un matériau expansé, et éventuellement dans laquelle l'élément d'étanchéité externe (113) comprend un agent antimicrobien et/ou antifongique.

8. Valve (1) selon une quelconque revendication précédente, dans laquelle uniquement l'élément de valve magnétique (14) et la bague ferromagnétique (5) ne sont pas élastiquement déformables.

9. Valve (1) selon une quelconque revendication précédente, dans laquelle le corps principal de valve (11) est effilé vers l'orifice de sortie (25).

10. Système de valve (100) comprenant une valve (1) selon une quelconque revendication précédente, un cathéter (16) couplé à l'orifice d'entrée (112) de la valve (1), et un mécanisme de retenue (28) couplé au cathéter (16), dans lequel le mécanisme de retenue (28) est conçu pour un déploiement dans une vessie.

11. Système de valve (100) selon la revendication 10, comprenant en outre :
un élément extensible (50) s'étendant à travers la valve (1) et le cathéter (16), et fixé à au moins une pièce du mécanisme de retenue (28) ; et
une pince de retenue (501) conçue pour entrer en prise avec l'élément extensible et empêcher un mouvement de celui-ci à travers le cathéter (16).

12. Système de valve (100) selon la revendication 10 ou 11, comprenant en outre un mécanisme de retenue externe (282) conçu pour empêcher une migration du système de valve (100) dans la vessie.

13. Système de valve (100) selon l'une quelconque des revendications 10 à 12, comprenant en outre un second cathéter (16) qui couple le mécanisme de retenue externe (282) à la valve (1).
